Europäisches Patentamt

European Patent Office

Office européen des brevets

(19)

(11) **EP 1 577 363 A1**

(12) **EUROPEAN PATENT APPLICATION**
published in accordance with Art. 158(3) EPC

(43) Date of publication:
**21.09.2005 Bulletin 2005/38**

(51) Int Cl.⁷: **C09K 5/16**, A61F 7/08

(21) Application number: **03782842.3**

(86) International application number:
**PCT/JP2003/016338**

(22) Date of filing: **19.12.2003**

(87) International publication number:
**WO 2004/061045 (22.07.2004 Gazette 2004/30)**

(84) Designated Contracting States:
**AT BE BG CH CY CZ DE DK EE ES FI FR GB GR
HU IE IT LI LU MC NL PT RO SE SI SK TR**

(30) Priority: **27.12.2002 JP 2002382632**

(71) Applicant: **Mycoal Products Corporation
Tochigi-chi, Tochigi 328-0067 (JP)**

(72) Inventors:
• **USUI, Kaoru Mycoal Products Corporation
Tochigi-shi, Tochigi 328-0067 (JP)**

• **KIMURA, Hisao Mycoal Products Corporation
Tochigi-shi, Tochigi 328-0067 (JP)**
• **AIDA, Michio Mycoal Products Corporation
Tochigi-shi, Tochigi 328-0067 (JP)**
• **DODO, Toshihiro Mycoal Products Corporation
Tochigi-shi, Tochigi 328-0067 (JP)**

(74) Representative: **Rupp, Christian et al
Mitscherlich & Partner
Patent- und Rechtsanwälte
Sonnenstrasse 33
80331 München (DE)**

(54) **EXOTHERMIC COMPOSITION AND EXOTHERMIC ELEMENT**

(57) In a heat generating composition which generates heat by contacting with air, a heat generating composition which is **characterized in that** an exothermic substance, a reaction promoter, water and a carbon component are essential components, water mobility value thereof is 20 or less, maximum particle size of water-insoluble solid components excluding the reaction promoter and water is 1 mm or less where 80% or more thereof has a particles size of 300 μm or less, water in the heat generating composition does not function as a barrier layer and exothermic reaction takes place when contacted to the air. A heat generating composition having a molding property and a shape-holding property and also having an exothermic characteristic whereby exothermic reaction is able to start by contacting to the air immediately after the molding and a heat generating body using the same are provided.

**Fig. 1**

EP 1 577 363 A1

**Description**

Technical Field

The present invention relates to a heat generating composition having an exothermic characteristic having a molding property and a shape-holding property and being able to start in exothermic reaction soon after contacting to air after molding and also to a heat generating body using the same.

Background Art

Heat generating compositions utilizing an oxidation reaction of metal such as iron have been provided as powdery, viscous and creamy substances. Heat generating bodies utilizing the same are very excellent in view of cost, safety, exothermic temperature, etc. and, for example, they have been already practically used as the so-called chemical warmers for human body being packed in a bag having gas permeability.

Seeking the prevention of uneven distribution of heat generating composition and the fitting property by means of various shapes for achieving more comfortable feeling in use, various heat generating compositions for attempting a shape holding property and also maintenance of exothermic characteristic using thickener, binder, etc. have been proposed. For example, in Japanese Patent Laid-Open No. 04/293,989, there are proposals for a method of manufacture of a heat generating composition where granulation is conducted so as to make the average particle size 0.5 mm or more and a method of manufacture of a heat generating composition where particle strength after granulation is enhanced by compounding of 10 to 20 parts by weight of a pressure-sensitive adhesive binder component with adding water.

In Japanese Patent Laid-Open No. 06/343,658, there is a proposal for a disposable body warmer comprising a heat generating composition endowed with a shape holding property by addition of a powdery thickener such as corn starch and potato starch.

In Japanese Patent Laid-Open No. 59/189,183, there is a proposal for a solid heat generating composition where a binder such as CMC is mixed with a powdery heat generating composition followed by subjecting to a compressive molding.

In WO 00/13626, there is a proposal for a heat generating body which is compressed and united by pressure using a cross-linking agent, etc. and a water-absorptive polymer.

In Japanese Patent Laid-Open No. 09/75,388, there is a proposal for a heat generating composition and a heat generating body in a form of ink or cream by endowing with viscosity using a thickener and also for a method of manufacturing the same.

In Japanese Patent Laid-Open No. 60/101,448, there is a proposal where surface of a molded product of a heat generating composition is covered with a gas-permeable film such as CMC using a binder to attempt maintenance of the shape.

In Japanese Patent Laid-Open No. 09/276,317 and in Japanese Patent Laid-Open No. 11/299,817, there are proposals that and heat generating composition is made into a viscous or creamy form and the shape is changed from the conventional rectangle to a trapezoid or an ellipse so as to adapt the outline of a substance to be warmed.

However, although those powdery heat generating compositions have a good exothermic characteristic, their exothermic property is not able to be fully exploited because for example distribution of exothermic temperature is not constant due to uneven location of the heat generating composition, feeling in actual use is bad and manufacture of a heat generating body having a shape meeting the shape of a substance to be warmed is difficult because the composition is filled in a container bag having air permeability and used as a heat generating body.

Further, when a powdery heat generating composition is filled in a bag-shaped receiver to manufacture a heat generating body, the material is intermittently moved so that the heat generating composition is fallen down during the material is stopped whereby there is a problem that manufacturing speed becomes slow because stop and movement of the material are frequently repeated.

Incidentally, there is a product where uneven distribution of a heat generating composition is prevented utilizing the vacuum upon heat generation by a porous membrane but it has not been able to completely prevent the uneven distribution.

Further, although a higher speed than a method of filling of powder is possibly achieved in a method where a heat generating composition is fallen down together with a movement of a material at a constant speed as will be noted in the manufacture of a sheet-shaped heat generating body, it is necessary to disperse a powdery heat generating body in unwoven fabric which is a substrate material and there is a limit for the manufacturing speed. There is a limit for the manufacture of heat generating bodies having various shapes as well.

On the other hand, in the case of a heat generating composition in a form of slurry, water content is high and maintenance of its shape is not possible as it is while, in the case of a viscous heat generating composition, fluidity is insufficient and it is not able to be directly molded but is to be subjected to a compression molding or the like.

In a viscous heat generating composition in a form of ink or cream where a thickener such as glue, acacia or CMC is added, it is excellent for prevention of uneven distribution, molding property and shape-holding property because of the use of a viscosity-endowing substance for bonding the particles of a heat generating body but its exothermic property is significantly bad. Similarly, in the case of a viscous heat generating composition prepared by the use of a thickener and a binder, it is excellent for prevention of uneven distribution, molding property and shape-holding property because of the use of a thickener and a binder for bonding the particles of a heat generating body but its exothermic property is significantly bad.

Thus, even when free water is absorbed with a support, a coating material, a water-absorbing material, etc., and heat-generating composition is viscous due to a binder, a thickener, an aggregation aid and a water-absorptive polymer. Therefore, the free water is not completely removed and the reaction becomes slow due to a bad affection of the thickener, etc. on the heat generating body whereby a quick temperature rise to a requested temperature and a heating for long time are difficult.

Further, drainage is bad in a creamy heat generating composition and, therefore, there are problems that long time is needed for absorption of free water with a support, etc. and that excessive free water remains in the heat generating composition and the free water inhibits the reaction. There is another problem that, when the amount of water to be added is reduced, time for reaction and heat generation becomes short. Accordingly, there is a problem in the manufacture of a heat generating body giving desired temperature and exothermic time in such respects that, although molding of a heat generating body of a super-slim type at a high speed is possible, a product which is able to generate the heat for long time is not able to be manufactured and that, when a heat generating body is made thick for extending the time for heat generation, free water is not completely removed and exothermic temperature rather lowers.

A heat generating composition where its shape is intended to be maintained using a binder, an excipient or a thickener is such a one where exothermic characteristic is greatly sacrificed.

The present inventors have found that an exothermic property significantly lowers when a heat generating composition containing a viscosity-endowing substance such as thickener, binder and aggregation aid in an amount of being able to create a shape-holding property has excessive water.

An object of the invention is to provide a heat generating composition where a heat generating body having any shape is able to be manufactured in any thickness and size using a molding means such as molding by stuffing, molding by passing through a die and slip casting, generation of powdery dust upon the manufacture of a heat generating body is able to be prevented, molding at high speed is possible and a heat generating body after molding has an excellent exothermic character and also to provide a heat generating body using the same.

Disclosure of the Invention

The present inventors have repeatedly conducted intensive studies for solving the above-mentioned problems and found that, when particle size of solid components and excessive water in a heat generating composition are made within appropriate ranges, heat generation is achieved without removal of water using a water-absorptive material or the like after molding, molding property, shape-holding property and exothermic characteristic are able to be highly maintained and a warming effect is achieved for a long period.

Thus, a heat generating composition of the invention is as mentioned in claim 1 that, in a heat generating composition which generates heat by contacting with air, a heat generating composition which is characterized in that an exothermic substance, a reaction promoter, water and a carbon component are essential components, water mobility value thereof is 20 or less, maximum particle size of water-insoluble solid components excluding the reaction promoter and water is 1 mm or less where 80% or more thereof has a particle size of 300 μm or less, water in the heat generating composition does not function as a barrier layer and exothermic reaction takes place when contacted to the air.

The heat generating composition mentioned in claim 2 is that according to claim 1, wherein particle size of all of the above water-insoluble solid components is 300 μm or less.

The heat generating composition mentioned in claim 3 is that according to claim 1, wherein the heat generating composition uses a heat generating composition having a water mobility value of 7 or more as a material and water content is adjusted by a non-oxidative gas.

The heat generating composition mentioned in claim 4 is that according to claim 1, wherein the heat generating composition contains at least one member selected from additional components consisting of water-retaining agent, water-absorptive polymer, hydrogen formation inhibitor, pH adjusting agent, surfactant, antifoaming agent, hydrophobic polymer compound, pyroelectric substance, far-infrared ray-radiating substance, negative ion-generating agent, antioxidant, aggregate, heat generating aid, oxidation catalyst, organosilicon compound, fibrous material, sanitary agent, fertilizer component, active aromatic compound, inactive aromatic compound, moisturizer and a mixture thereof.

A heat generating body mentioned in claim 5 is characterized in that at least a part of the heat generating composition mentioned in claim 1 is sealed in a container bag where at least a part thereof has air permeability.

The heat generating body mentioned in claim 6 is that according to claim 5, wherein the heat generating composition is layered and received in a container bag, the layered heat generating composition forms two or more plural sectional exothermic parts being separately located and an aggregated exothermic part is formed from aggregation of the sectional exothermic parts.

The heat generating body mentioned in claim 7 is that according to claim 6, wherein the container bag comprises a substrate material and a covering material, at least one of the substrate material and the covering material has gas permeability and each of the sectional exothermic parts is sectioned by a sectional part by means of a heat seal of the substrate material and the covering material.

The heat generating body mentioned in claim 8 is that according to claim 5, wherein the container bag comprises a substrate material and a covering material, at least one of the substrate material and the covering material has gas permeability, the heat generating composition is placed on the substrate material, a pressure-sensitive adhesive layer is further located at least on the heat generating composition and a spreading material is furthermore located thereupon.

The heat generating body mentioned in claim 9 is that according to claim 8, wherein at least the heat generating composition is subjected to a compressing treatment in the substrate material, covering material, gas permeable pressure-sensitive adhesive layer, spreading material and heat generating composition.

The heat generating body mentioned in claim 10 is that according to claim 7, wherein a perforation is formed on the sectional part.

The heat generating body mentioned in claim 11 is that according to claim 5, wherein any one or more of letters, designs, symbols, numerals, patterns, photographs and pictures are formed on at least on a part thereof including a releasing paper.

The heat generating body mentioned in claim 12 is that according to claim 5, wherein at least a part thereof including a releasing paper is colored.

The heat generating body mentioned in claim 13 is that according to claim 7, wherein a pressure-sensitive adhesive layer or a jell layer is layered on at least a part of exposed surfaces of at least one of the substrate material and the covering material.

The heat generating body mentioned in claim 14 is that according to claim 13, wherein the pressure-sensitive adhesive layer or the jell layer contains or carries at least one member selected from additional components consisting of moisturizer, negative ion-generating substance, bamboo carbon, pyroelectric substance, far-infrared ray-radiating substance, active aromatic compound, inactive aromatic compound, sanitary agent and a mixture thereof.

The heat generating body mentioned in claim 15 is that according to claim 5, wherein the heat generating body is intervened between two non-gas-permeable films or sheets, the two films or sheets are punched into a size which is not smaller than the heat generating body together with or after the intervening and the two films or sheets are adhered by fusion at the surrounding which is more than the size of the heat generating body together with or after the punching.

Brief Description of the Drawings

Fig. 1 is an oblique view of an embodiment of the heat generating body of the invention.
Fig. 2 is a cross-sectional view along the line Z-Z of the above.
Fig. 3 is a cross-sectional view of another embodiment of the heat generating body of the invention.
Fig. 4 is a cross-sectional view of another embodiment of the heat generating body of the invention.
Fig. 5 is a cross-sectional view of another embodiment of the heat generating body of the invention.
Fig. 6 is a cross-sectional view of another embodiment of the heat generating body of the invention.
Fig. 7 is a plane figure of another embodiment of the heat generating body of the invention.
Fig. 8 is a cross-sectional view along the line Y-Y of the above.
Fig. 9 is a plane figure of another embodiment of the heat generating body of the invention.
Fig. 10 is a plane figure of another embodiment of the heat generating body of the invention.
Fig. 11 is a cross-sectional view along the line X-X of the above.
Fig. 12 is a cross-sectional view of another embodiment of the heat generating body of the invention.
Fig. 13 is a scheme of a molding by passing through a die of the heat generating body of the invention using a scraper.
Fig. 14 is an illustrative view near the scraper.
Fig. 15 is an illustrative view near a pushing scraper in a molding of the heat generating body of the invention passing through a die using a pushing scraper.

Fig. 16 is an illustrative view showing a method for measurement of water mobility value in the invention.
Fig. 17 is an illustrative view showing a method for measurement of water mobility value in the invention.
Fig. 18 is an illustrative view showing a method for measurement of water mobility value in the invention.
Fig. 19 is an illustrative view showing a method for measurement of water mobility value in the invention.
Fig. 20 is an illustrative view showing a method for measurement of water mobility value in the invention.

Best Mode for Carrying Out the Invention

The heat generating composition of the invention is a heat generating composition where particle size of water-insoluble solid components except a reaction promoter and water is made a predetermined value or less, excessive water having 20 or less water mobility value is contained, shape of a layered substance molded by molding such as molding by passing a die, molding by stuffing and molding by casting is maintained and generation of heat is made possible without removal of water such as water absorption and dehydration using a substrate material after the molding. Accordingly, it is not necessary to make a container bag water-absorptive but a heat generating body is able to be prepared by receiving a non-water-absorptive container bag.

Thus, in the heat generating composition of the invention, water is used as a binder and the components are bonded by surface tension of water existing among the components whereby fluidity, molding property, shape-holding property and exothermic characteristic are highly maintained and that is entirely different from the conventional viscous heat generating composition where the components are bonded by an adhesive or the like, exothermic characteristic is sacrificed and only molding property and shape-holding property are persuaded. The heat generating composition of the invention is a heat generating composition where molding property and shape-holding property are able to be maintained while an exothermic characteristic is still brought out and, when it is used, a layered heat generating composition and a heat generating body having an excellent exothermic characteristic in various sizes, various thicknesses and various sizes are provided.

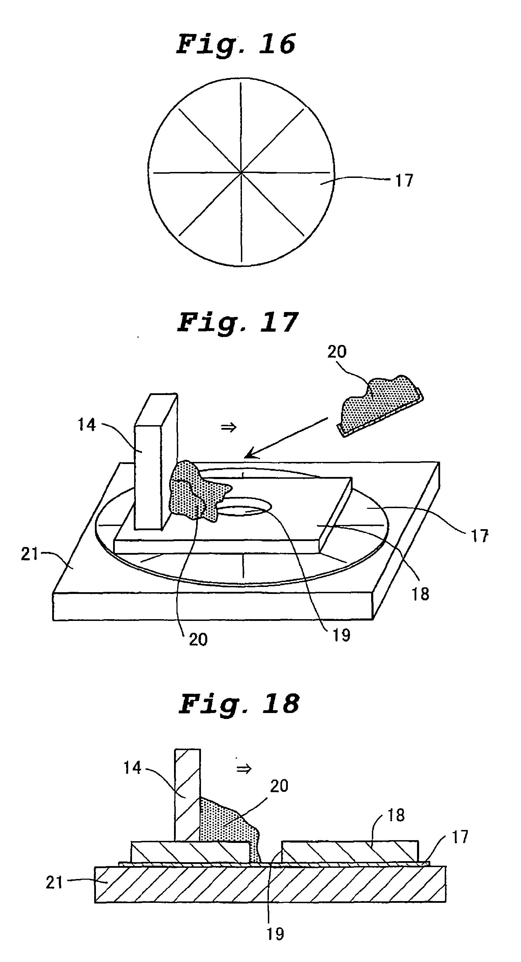

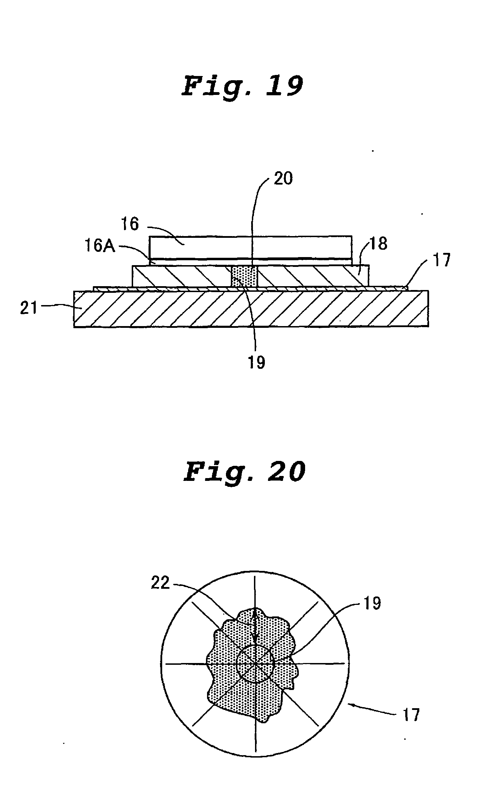

The water mobility value used here means a value showing an amount of excessive water in the heat generating composition which is able to be moved to the outside of the heat generating composition. The water mobility value will be illustrated using Fig. 16 to Fig. 20. As shown in Fig. 16, a filter paper 17 of No. 2 where eight lines are drawn radiating from the central point with an interval of 45° is placed on a stainless steel plate 21 as shown in Fig. 17 and Fig. 18, a template 18 of 150 mm length and 100 mm width having a hollow cylindrical hole 19 of 20 mm inner diameter and 8 mm height is placed at the center of the filter paper 17, a sample 20 is placed near the hollow cylindrical hole 19, a stuffer plate 14 is moved on and along the template 18, the sample 20 is placed into the hollow cylindrical hole 19 together with stuffing and the sample is cut by abrasion along the surface of the template 18 (stuff molding). After that, a non-water-absorptive polyethylene film 16A of 70 µm is placed so as to cover the hole 19 and a flat plate 16 made of stainless steel having 5 mm thickness, 150 mm length and 150 mm width is further placed thereon and kept for 5 minutes so as not to cause an exothermic reaction. After that, the filter paper 17 is taken out (Fig. 20) and an oozed-out locus of water or aqueous solution is read as the distance 22 from the circumference which is a rim of the hollow cylindrical hole 19 in a mm unit along the radiating lines. Similarly, the distance 22 from each line is read and eight values in total are obtained. Each of the eight values (a, b, c, d, e, g and h) read out as such is defined as a measured value of water content.

An arithmetic mean value of the eight measured values is defined as the water content (mm) of the sample.

In order to measure the real water content value, a water content value is defined as a compounded water content value of the heat generating composition corresponding to the weight of the heat generating composition of 20 mm inner diameter and 8 mm height, similar measurement was conducted only with water corresponding to such a water content amount and the result calculated similarly is defined as the real water content amount (mm). Value obtained by dividing the water content value by the real water content value followed by multiplying with 100 is a water mobility value.

Thus,

$$\text{Water mobility value} = [(\text{Water content value (mm)}) / (\text{Real water content value (mm)})] \times 100.$$

Incidentally, the water mobility value is a value upon layering by a molding by stuffing, etc.

The water mobility value (0 to 100) of the heat generating composition of the invention is usually 20 or less, preferably 3 to 18 and, more preferably, 5 to 15. When it is more than 20, an exothermic characteristic is deteriorated unless the excess water is removed from the molded heat generating composition by means of water absorption or dehydration after the molding.

With regard to particle size of the water-insoluble solid component, separation is conducted using a sieve and particle size of the component passing through the sieve is calculated from the diameter of the sieve. Thus, sieves of 8, 12, 20, 32, 42, 60, 80, 100, 115, 150, 200, 250 and 280 meshes and a receiving dish are combined in this order from up to down. Water-insoluble solid component particles (about 50 g) are placed on the uppermost sieve of 8 meshes and shaken for 1 minute using an automatic shaker. Weights of the water-insoluble solid component particles on each of the sieves and the receiving dish are weighed. The total amount thereof is defined 100% and particle size distribution is determined from weight fractions. When the sum of all receiving dishes under the sieve of a specific mesh size becomes 100% which is the sum of the above particle size distribution, the size (μm) calculated from the diameter of the specific mesh is defined as the particle size of the water-insoluble solid component.

Incidentally, each of the mesh sieves may be compounded with other mesh sieves.

Here, it is defined that particles passing through a 16-mesh sieve correspond to particle size of 1 mm or less, those passing through a 20-mesh sieve correspond to particle size of 850 μm or less, those passing through a 48-mesh sieve correspond to particle size of 300 μm or less, those passing through a 60-mesh sieve correspond to particle size of 250 pm or less, those passing through a 65-mesh sieve correspond to particle size of 212 μm or less, those passing through a 80-mesh sieve correspond to particle size of 180 μm or less, those passing through a 100-mesh sieve correspond to particle size of 150 μm or less, those passing through a 115-mesh sieve correspond to particle size of 125 μm or less, those passing through a 150-mesh sieve correspond to particle size of 100 μm or less and those passing through a 250-mesh sieve correspond to particle size of 63 μm or less.

The perforation at the sectioned part of the heat generating body includes that which is intermittently cut for improving the bending property of the sectioned part and that which is intermittently cut so that cutting by hand is possible. The perforation may be formed in all sections or formed partially.

Incidentally, a molding property means that, by means of a molding by passing through a mold using a trimming die having a trimming hole and a slip casting using a casting mold, a layered product of a heat generating composition is prepared by the shape of the trimming hole or the casting die and a shape-holding property means that the molded layered product is covered at least by a covering material and the shape is able to be maintained until a sealed part is formed between the substrate material and the covering material. It is at least necessary that surrounding part of the heat generating composition is not collapsed and a sealing part is able to be formed without the so-called "spots" in the sealing part. The presence of the spots causes insufficient sealing.

Principle for heat generation of the heat generating body is that heat generation upon oxidation of metal powder which is an exothermic substance is utilized. Such an oxidation reaction is particularly affected by water content and the reaction is significantly slow when the water content is too much or too little and appropriate water content is necessary for initiating the heat generation and maintaining it. It is further necessary for achieving the shape-holding property that particle size of water-insoluble solids except reaction promoter and water in the heat generating composition is made as small as possible and that particles are held by means of surface tension by excessive water.

The heat generating composition has been developed as a result of a conclusion that, in order to result in the exothermic reaction, it is better that the excessive water is to be removed as much as possible and, when the excessive water is made as little as possible, heat generation is able to be started efficiently.

Thus, the heat generating composition of the invention is that, in a heat generating composition which generates heat by contacting to the air, an exothermic substance, a reaction promoter, water and a carbon component are essential components, water mobility value thereof is 20 or less and maximum particle size of water-insoluble solid components excluding the reaction promoter and water is 1 mm or less where 80% or more thereof has a particle size of 300 μm or less to give a heat generating composition having a molding property whereby particle size of water-insoluble solid components and excessive water are adjusted to within appropriate ranges, molding property and shape-holding property are very good, layering by means of molding by passing through a mold, molding by stuffing, slip casting, etc. is easy, heat generating body of an ultra-thin type to a thick type is able to be manufactured at a high speed, the heat generating composition is able to be uniformly distributed in a packing material and an exothermic reaction is able to be started even when the excessive water is not removed by an absorptive material or the like.

The heat generating composition of the invention is applied to a heat generating body which generates heat by contacting to oxygen in the air. In addition, the invention is not only used for warming of human body but also is applied to a heat generating body which is used for warming of pets and machines.

The above heat generating composition may be also compounded with at least one member selected from additional components consisting of water-retaining agent, water-absorptive polymer, hydrogen formation inhibitor, pH adjusting agent, surfactant, antifoaming agent, hydrophobic polymer compound, pyroelectric substance, far-infrared ray-radiating substance, negative ion-generating agent, antioxidant, aggregate, heat generating aid, oxidation catalyst, organosilicon compound, fibrous material, sanitary agent, fertilizer component, active aromatic compound, inactive aromatic compound, moisturizer and a mixture thereof.

If necessary, at least one member selected from water-soluble macromolecular substance, thickener, binder, excipient, aggregating agent and soluble viscous material may be compounded therewith within such an extent that the exothermic property is not deteriorated.

With regard to a heat generating body using the above-mentioned heat generating composition, an example thereof is a heat generating body which is constituted in such a manner that the heat generating composition is layered between a gas-permeable packing material constituting a covering material and a substrate material.

By means of a molding by passing through a die using a trimming die, a molding by stuffing and a slip casting using a casting die, it is now possible to homogeneously distribute in a packing material at high speed and to easily manufacture layered product of heat generating composition and heat generating body in a desired shape from an ultra-thin type to a thick type.

It is also possible to use a magnet for molding of the heat generating composition of the invention. When a magnet is used, it is now easy to receive the heat generating composition into a die and to release the molded product from the die whereby molding of the layered heat generating composition is easier.

In the heat generating body of the invention, the above-mentioned heat generating composition is received in a container bag where at least a part thereof is permeable to gas. The container bag is constituted from substrate material and covering material and at least one of the substrate material and the covering material or a part of at least thereof is permeable to gas. With regard to a layered heat generating composition, one thereof may forms an exothermic part or two or more plural ones are separately arranged to form sectional exothermic part and, further, an aggregated exothermic part may be formed from aggregation of the sectional exothermic parts. The container bag may be also constituted from a packing material including a spreading material besides the packing material such as the substrate material and the covering material.

The layered heat generating composition may be in any form and its examples in the case of two-dimensional form are circle, ellipse, rectangle, square, triangle, rhombus and trapezoid while its examples in the case of three-dimensional form are disk shape, triangular pyramid, pyramid, cone, sphere, rectangular parallelepiped, regular hexahedron, cylinder and long ellipsoid.

Although there is no particular limitation for the size of the layered heat generating composition, flexibility is available in the heat generating body as a whole when the size is as small as possible in case the above-mentioned sectional exothermic part is formed. Thus, although the numbers of the above sectional heat generating body may be made 10 or more per heat generating body, they are usually made 3 to 9 sections in the case of heat generating body of a size of 100 mm width and 120 mm length in view of duration of heat generating time. Thus, a heat generating body comprising 3 to 9 sectional exothermic parts is formed.

Each one of the above-mentioned sectional exothermic parts may be sectioned by sectioning parts by means of sealing of the gas-permeable packing material.

With regard to the seal of the sectioned part, its examples are that, around the sectional exothermic part, the seal is a point adhesion (intermittent adhesion), heat seal, point adherence, adhering seal or a combination thereof between the substrate material and the covering material thereof.

It is also possible to form perforation in the above sectioned part.

With regard to the gas-permeable packing material, an example thereof is a gas-permeable packing material where nonwoven fabric is laminated on perforated film or porous film.

When the above packing material is not permeable to gas, it is possible to form a pressure-sensitive adhesive layer or a jell layer.

It is also possible that at least the above layered heat generating composition and surrounding thereof are covered by a gas-permeable pressure-sensitive adhesive layer so that movement of the layered product between the substrate material and the covering material is prevented. It is further possible that a spreading material such as nonwoven fabric is formed between the gas-permeable pressure-sensitive adhesive layer and the covering material.

Among the above-mentioned substrate material, covering material, gas-permeable pressure-sensitive adhesive layer, spreading material and heat generating composition, at least the heat generating composition may be subjected to a compressing treatment.

Thus, in a product where a layered product which is a molded product of the heat generating composition of the invention is appropriately compressed by pressure, there is a significant improvement in a shape-holding property. For example, even when a perforated film where adjustment of pressure is difficult is used as a material for the gas-permeable part in place of a porous film or when inner pressure of the container bag becomes higher than the outer pressure, loss of the shape hardly takes place and a perforated film is still able to be used. Accordingly, latitude in the selection of gas-permeable material becomes wide, reduction in cost is able to be done and the object to be warmed is able to be uniformly warmed at an appropriate temperature for long time.

Whole or a part of the surface of the outer layer part of the heat generating composition may be made uneven. It is also possible that whole or a part of the surface of the outer layer part of the heat generating composition or a material on which the composition is layered is made uneven.

In the exothermic part, at least a part thereof or one sectional exothermic part may contain a magnetic substance.

In the heat generating body, any one or more of letters, designs, symbols, numerals, patterns, photographs and pictures may be formed on at least a part thereof including a releasing paper.

In the above heat generating body, at least thereof including a releasing paper may be colored.

In the above substrate material or covering material, at least a part of at least one of exposed surfaces may be equipped with a pressure-sensitive adhesive layer or a jell layer.

The above pressure-sensitive adhesive layer may be arranged in a form of parallel stripes continuously extending from upper rim to lower rim between the above plural sectional exothermic parts.

The pressure-sensitive adhesive layer or the jell layer may contain or carry at least one member selected from additional components consisting of moisturizer, negative ion-generating substance, bamboo carbon, pyroelectric substance, far-infrared ray-radiating substance, active aromatic compound, inactive aromatic compound, sanitary agent and a mixture thereof.

The above heat generating body is received in a non-container bag having air permeability, stored and transported and an example thereof is that the heat generating body is intervened between two non-gas-permeable films or sheets, the two films or sheets are punched into a size which is not smaller than the heat generating body together with or after the intervening and the two films or sheets are adhered by fusion at the surrounding which is more than the size of the heat generating body together with or after the punching.

The heat generating body prepared as such is used not only for warming in winter time but also for the diseases such as muscle stiffness of the shoulder, muscular pain, muscular stiffness, lumbago, chill in hands and feet, neuralgia, rheumatism, bruises and sprain and a therapeutic effect by warming can be sufficiently expected. It is able to be further utilized for heating and warming of machines and pets and also for deoxidizers and antifungal agents.

With regard to other uses of the heat generating body, an example is that the heat generating body is applied to the pain site of the body of a person who needs a treatment and skin temperature and maintaining time are appropriately selected depending upon the person needing a treatment so that pain is substantially mitigated pleasantly whereby acute, recurrent or chronic pain of muscle or skeleton or related pain is treated.

In the heat generating composition of the invention, although there is no particular limitation for the compounding ratio thereof, an example is that 1.0 to 50 part(s) by weight of carbon component, 1.0 to 50 part(s) by weight of reaction promoter, 0.01 to 20 part(s) by weight of water-absorptive polymer, 0.01 to 10 part (s) by weight of water-retaining agent, 0.01 to 5 part(s) by weight of pH adjusting agent and an appropriate amount of water to 100 parts by weight of an exothermic substance.

To the above heat generating composition may be further added the following substances in the following compounding rates in terms of to 100 parts by weight of the exothermic substance.

Thus, they are 0.01 to 5 part(s) by weight of hydrogen formation inhibitor, 0.01 to 5 part (s) by weight of surfactant, 0.01 to 5 part(s) by weight of antifoaming agent, each 0.01 to 5 part(s) by weight of hydrophobic polymer compound, aggregate, fibrous material, pyroelectric substance, far-infrared ray-radiating substance, negative ion-generating substance and organosilicon compound, each 0.01 to 10 part(s) by weight of moisturizer, sanitary agent, fertilizer component and exothermic acid and each 0.01 to 0.1 part by weight of water-soluble polymer, thickener, binder, excipient, aggregating agent and soluble pressure-sensitive adhesive material.

The maximum particle size of solid components excluding reactor promoter and water among the components constituting the heat generating composition is 1 mm or less, preferably 500 μm or less, more preferably 300 μm or less, still more preferably 250 μm or less and, particularly preferably, 200 μm or less.

Among the components constituting the heat generating composition, particle size of 80% or more of water-insoluble solid components excluding reaction promoter and water is 300 μm or less, preferably 250 μm or less and, more preferably, 200 μm or less.

Incidentally, molding property and shape-holding property of the heat generating composition are dependent upon the particle size of the water-insoluble solid components except reaction promoter and water and the smaller the particle size, the better.

With regard to the above exothermic substance, anything may be used so far as it generates heat when contacted to the air although, generally, metal is used. For example, iron powder, zinc powder, aluminum powder or magnesium powder, alloy powder containing one or more member(s) of the metals as such and a mixed metal powder containing one or more member(s) thereof may be used and, among those metal powders, it is desirable to use iron powder which is best in view of an overall viewpoints for safety, handling property, cost, preserving property and stability. With regard to the iron powder, it is possible to use cast iron powder, atomized iron powder, electrolyzed iron powder and reduced iron powder. Further, the iron powder as such may contain carbon and an iron alloy containing 50% or more of iron may be used as well. With regard to the type of metal used for such an alloy, there is no particular limitation so far as the iron component acts as a component for the heat generating composition and its examples are manganese, copper and nickel.

Here, the iron powder may contain oxygen and/or carbon component(s) and/or may be coated with the same.

Iron powder where the surface is partially coated with 0.3 to 3.0% by weight of a conductive carbonic substance is particularly useful. Examples of the conductive carbonic substance are carbon black and active carbon while examples of the iron powder are reduced iron powder, atomized iron powder and sponge iron powder and the case where the conductive carbonic substance is active carbon and the iron powder is reduced iron powder is particularly useful for a heat generating body.

In that case, with regard to a method for coating the iron powder with a carbonic component, a cathode thin film is able to be formed by a coating treatment for 30 minutes to 3 hours using a ball mill, a conical blender or the like. An example is a method where a mixer of a compressing type (such as AM-15F manufactured by Hosokawa Micron) is used, 0.1 to 10 part(s) by weight of carbon component is used to 100 parts by weight of iron powder and kneading is conducted at 500 to 1, 500 rpm for 10 to 80 minutes.

Although there is no particular limitation for the amount of oxygen contained in iron so far as there is no affection on the exothermic characteristic, it is preferred that the oxygen amount in the iron component as a material before mixing and the oxygen amount in the iron component in a heat generating composition after layering of a heat generating composition are 23% by weight or less. More preferably, the amount is 15% by weight or less and, still more preferably, that is 10% by weight or less. The oxygen amount in the iron component as a material and the oxygen amount in the iron component in a heat generating composition after layering of a heat generating composition may be different. Incidentally, the oxygen amount in the iron component in a heat generating composition after layering is measured in such a manner that, in a nitrogen atmosphere, a heat generating composition after layering is placed in deionized water which was substituted with nitrogen and stirred and the iron component is separated using a magnet and dried in a nitrogen atmosphere to determine an oxygen amount in the iron component.

Examples of the above carbon component are carbon black, graphite and active carbon. Although active carbon prepared from coconut shell, wood, charcoal, coal, bone black, etc. is useful, that which is prepared from other materials such as animal product, natural gas, fat, oil and resin is also useful for a heat generating composition of the invention. Although there is no limitation for the type of the active carbon used, an active carbon having an excellent adsorption-retaining ability is more preferred. With regard to the property of the carbon component, it is preferred that iodine adsorption ability is 550 to 1,200 mg/g and decolorizing ability for Methylene Blue is 60 to 300 mg/g and, more preferably, iodine adsorption ability is 800 to 1,200 mg/g and decolorizing ability for Methylene Blue is 100 to 300 mg/ g. In the invention, a mixture of the above-mentioned carbon substances may be used as well.

With regard to the above reaction promoter, anything may be used so far as it is able to promote the reaction of an exothermic substance. Examples thereof are metal halide such as sodium chloride, potassium chloride, magnesium chloride, calcium chloride, ferrous chloride, ferric chloride, cupric chloride, manganese chloride and cuprous chloride; metal sulfate such as potassium sulfate, sodium sulfate, magnesium sulfate, calcium sulfate, copper sulfate, ferrous sulfate, ferric sulfate and manganese sulfate; nitrate such as sodium nitrate and potassium nitrate; acetate such as sodium acetate; and carbonate such as ferrous carbonate. Each of them may be used solely or may be used in combination.

Usually, such an oxidation promoter is used as an aqueous solution although the powder as it is may be used as well.

With regard to the above water, that which is from an appropriate source may be used. There is no limitation for purity, type, etc. thereof.

With regard to the above water-absorptive polymer, there is no particular limitation so far as it has water-retaining property and water-absorptive property to water or to an aqueous solution. Preferably, it is to be insoluble in water or in an aqueous solution of 50°C or lower and, when heat generating time, etc. are taken into consideration, the absorptive capacity as a water-absorptive amount of a 11% aqueous saline solution is preferred to be 5 g/g or more.

When the above water-absorptive polymer is a mixture of a complex of resins having different water-absorbing speed, there is no limitation for the ratio of the water-absorbing speed of the water-absorptive resin having the quickest water-absorbing speed and that of the water-absorptive resin having the slowest water-absorbing speed although it is preferred to be three-fold or more.

Examples of the above water-absorptive polymer are polyacrylate type, polyvinyl alcohol type, polyvinyl alcohol/acrylate copolymer, isobutyrene/anhydrous maleate type copolymer, N-vinylacetamide type, N-alkylacrylamide type copolymer and starch/acrylate type copolymer. To be more specific, preferred examples are poly(meth)acrylic acid derivative such as alkyl polyacrylate, sodium (meth) acrylate-vinyl alcohol copolymer (saponified product of methyl (meth)acrylate-vinyl acetate copolymer), saponified product of poly(meth)acrylonitrile type polymer; cellulose derivative such as isobutyrene-maleate type copolymer and alkali metal salt of carboxymethyl cellulose; alginic acid derivative such as polyacrylamide and sodium alginate or propylene glycol alginate; starch derivative such as starch sodium glycolate, sodium salt of starch phosphate and starch-acrylate graft copolymer; poly-N-vinylacetamide derivative such as N-vinylacetamide polymer; polyvinyl alcohol derivative such as N-alkylacrylamide type copolymer polyvinyl alcohol, polyvinylformal and polyvinylacetal; and others such as PVA type-acrylate type copolymer, isobutyrene-maleic acid copolymer and a cross-linked product thereof, cross-linked product of vinyl alcohol-acrylate (carboxyl group-containing substance) copolymer, neutralized product of polyacrylic acid type of a self-cross-linking type, cross-linked product of acrylate type copolymer, cross-linked product of partially neutralized product of polyacrylic acid, cross-linked product of polyacrylate, saponified product of acrylate-vinyl acetate copolymer and a cross-linked product thereof, cross-linked product of acrylic acid salt-acrylic acid ester copolymer, cross-linked product of acrylate-acrylamide copolymer, cross-linked product of hydrolyzed product of acrylamide copolymer, cross-linked product of copolymer of 2-acrylamide-2-methylpropanesulfonic acid with acrylic acid, hydrolyzed product of cross-linked product of polyacrylonitrile, cross-linked product of hydrolyzed product of acrylonitrile copolymer, cross-linked product of hydrolyzed product of starch-acrylic acid copolymer, cross-linked product of starch-acrylonitrile copolymer and cross-linked product of hydrolyzed product thereof, cross-linked product of saponified product of vinyl ester-ethylene type unsaturated carboxylic acid copolymer, cross-linked product of vinyl alcohol-anhydrous maleate (cyclic anhydrous product) copolymer, N-vinylacetamide polymer or copolymer, cross-linked product of carboxymethyl cellulose salt (partially cross-linked product of polysaccharide), cross-linked product of cationic monomer, cross-linked product of polyethylene oxide, polyethylene oxide which is cross-linked with acrylic acid, cross-linked polyalkylene oxide, cross-linked product of copolymer of methoxypolyethylene glycol with acrylic acid, cross-linked product of polyacrylate, cross-linked product of starch-acrylic acid graft copolymer, hydrolyzed product of cross-linked product of starch-acrylonitrile graft copolymer, hydrolyzed product of acrylate-vinyl acetate copolymer, cross-linked product of acrylate-acrylamide copolymer and hydrolyzed product of cross-linked product of polyacrylonitrile. Other examples are polyethylene oxide cross-linked with acrylic acid, cross-linked product of sodium cabroxycellulose, anhydrous maleate-isobutyrene and a product where comonomer such as maleate, itaconate, 2-acrylamid-2-methylsulfonate, 2-acryloylethanesulfonic acid or 2-hydroxyethyl acrylate is copolymerized with acrylic acid.

Examples of a water-absorptive polymer having biodegradability are cross-linked product of polyethylene oxide, cross-linked product of polyvinyl alcohol, cross-linked product of carboxymethyl cellulose, cross-linked product of alginic acid, cross-linked product of starch, cross-linked product of polyamino acid and cross-linked product of polylactic acid.

With regard to the above water-retaining agent, there is no particular limitation so far as it is able to retain water and examples thereof are porous material derived from plants having high capillary function and hydrophilic property such as wood powder, pulp powder, active carbon, saw dust, cotton cloth having many cotton fluffs, short fiber of cotton, paper dust and plant material, clay mineral of aqueous magnesium silicate type such as active clay and zeolite, pearlite, vermiculite and silica type porous substance, coralline stone and substance of a volcanic ash type (such as terraballoon, *shirasu* balloon and *taisetsu* balloon).

Wood powder, terraballoon, *shirasu* balloon and *taisetsu* balloon are particularly preferred. More preferred one is that where particles having a size not more than 250 µm occupies 50% or more and still more preferred one is that where particles having a size not more than 150 µm occupies 50% or more.

In order to increase the water-retaining property and to enhance the shape-holding property of such a water-retaining agent, the agent may also be subjected to a processing treatment such as burning and/or disintegration.

Further, if desired, diatomaceous earth, alumina, cellulose powder or the like may be added to the heat generating composition of the invention. It is also possible to add a preventer for solidification thereto.

With regard to the above pH adjusting agent, weak acid salt of alkali metal, hydroxide, etc. or weak acid salt of alkaline earth metal, hydroxide, etc. are listed and examples thereof are $Na_2CO_3$, $NaHCO_3$, $Na_3PO_4$, $Na_2HPO_4$, $Na_5P_3O_{10}$, $NaOH$, $KOH$, $CaCO_3$, $Ca(OH)_2$, $Mg(OH)_2$, $Ba(OH)_2$, $Ca_3(PO_4)_2$ and $Ca(H_2PO_4)_2$.

With regard to the above hydrogen suppressor, anything may be used so far as it suppresses generation of hydrogen and examples thereof are that which comprises at least one or more member(s) selected from the group consisting of metal sulfide such as calcium sulfide, sulfite such as sodium sulfite, thiosulfate such as sodium thiosulfate, oxidizing agent, alkaline substance, sulfur, antimony, selenium, phosphorus and tellurium and the above-mentioned pH adjusting agent. When it is previously mixed with metal powder which is an exothermic agent, adding amount is able to be reduced and that is more effective.

With regard to the above oxidizing agent, nitrate, nitrite, oxide, peroxide, halogenated oxygen acid salt, permanganate and chromate may be listed and examples thereof are $NaNO_3$, $KNO_3$, $NaNO_2$, $KNO_2$, $CuO$ and $MnO_2$.

Examples of the above alkaline substance are silicate, borate, secondary phosphate, tertiary phosphate, sulfite, thiosulfate, carbonate, bicarbonate, $Na_2SiO_3$, $Na_4SiO_4$, $NaBO_4$, $Na_2B_4O_7$, $KBO_2$, $Na_2HPO_4$, $Na_2SO_3$, $K_2SO_3$, $Na_2S_2O_3$, $Na_2CO_3$, $NaHCO_3$, $K_2S_2O_3$, $CaS_2O_3$, $Na_3PO_4$ and $Na_5P_3O_{10}$.

In case the above hydrogen suppressors are used in combination, examples thereof are a combination of alkali weak acid salt-alkali weak acid salt such as $Na_2SO_3$-$Na_2SiO_3$, $Na_2SO_3$-$Na_2SiO_3$, $Na_2SO_3$-$Na_2B_4O_7$, $Na_2SO_3$-$Ca(OH)_2$, $Na_2B_4O_7$-$Na_3PO_3$ and $Na_2CO_3$-$Na_2SO_3$; and a combination of oxidizing agent-alkali weak acid salt such as $Na_3PO_4$-$Na_2SO_3$, S-$Na_2SO_3$ and S-$Na_2S_2O_3$.

Amount of the hydrogen suppressor used in terms of the total amount of each hydrogen suppressor to iron powder is preferably 0.01 to 12.0% by weight, more preferably 0.05 to 8% by weight and, still more preferably, 0.5 to 2.0% by weight. When it is less than 0.01% by weight, a suppressing effect for generation of hydrogen is poor while, when it is more than 12.0% by weight, although there is a suppressing effect for generation of hydrogen, exothermic temperature lowers and that is not suitable.

With regard to a method for addition, it is preferred to add as an aqueous solution in view of workability and uniformity of mixing but, even when it is added as a solid separately from water, there result is nearly the same as in the case of an aqueous solution for a suppressive effect for hydrogen.

With regard to the above far-infrared ray-radiating substance, anything may be used so far as it radiates far-infrared ray and examples thereof are ceramic, alumina, zeolite, zirconium and silica. One of them may be used solely or two or more thereof may be used as a mixture.

With regard to the above negative ion-generating substance, anything may be used so far as it generates minus ion as a result either directly or indirectly and examples thereof are ferroelectric substance such as tourmaline, granite, Rochelle salt, glycine sulfate, phosphorus potassium phosphate and calcium strontium propionate; exciting agent such as minus ionized Si, $SiO_2$, Davis ore, brannerite and feldspar; and ore containing radioactive substance such as radon. One of them may be used solely or two or more thereof may be used as a mixture. Those having hydroxyl group is present together or is retained by a substrate material are more effective.

With regard to the above pyroelectric substance, there is no limitation so far as it has pyroelectricity. Examples thereof are tourmaline, hemimorphic ore and pyroelectric ore. Tourmaline or achroite which is a kind of tourmaline is particularly preferred. Examples of tourmaline are dravite, schorl and elbaite.

With regard to the above fertilizer component, it is possible to use natural fertilizer such as bone powder and mineral fertilizer and chemical fertilizer such as urea, ammonium sulfate, ammonium chloride, calcium perphosphate, calcium biperphosphate, potassium chloride, potassium sulfate, calcium chloride and calcium sulfate either solely or in a form of a compounded fertilizer where the above-mentioned ones are mixed in an appropriate compounding ratio although that where three elements of nitrogen, phosphate and potassium are appropriately contained is preferred. It is also possible to add wood charcoal, ash, etc. having an action of controlling the growth of miscellaneous bacteria, an action of improving the soil neutralization and other effects.

With regard to the above exothermic aid, it includes metal powder, metal salt, metal oxide, etc. and its examples are Cu, Mn, $CuCl_2$, $FeCl_2$, $FeCl_3$, $CuSO_4$, $FeSO_4$, CuO, manganese dioxide, cupric oxide and triiron tetroxide as well as a mixture thereof.

With regard to the above hydrophobic polymer compound, anything may be used so far as it is a macromolecular compound where contact angle to water is 40° or more, more preferably 50° or more and, still more preferably, 60° or more for making drainage in the composition good. There is also no particular limitation for its shape and examples thereof are powder, granule, particle and tablet where preferred ones are powder, particle and granule.

Other examples are polyolefin such as polyethylene and polypropylene; polyester such as polyethylene terephthalic acid; and polyamide such as nylon.

With regard to the above organosilicon compound, anything including monomer, lowly-condensed product and polymer may be used so far as it is a compound having at least Si-O-R and/or Si-N-R and/or Si-R bond(s) and examples thereof are organosilane compound such as methyl trialkoxysilane (e.g. methyl triethoxysilane) and tetraalkoxysilane (e.g., tetraethoxysilane); polyorganosiloxane (polysiloxane resin) such as dimethyl silicone oil and diphenyl silicone oil; cyclic siloxane such as hexaorganocyclotrisiloxane; and a silicone resin composition containing the same.

With regard to the above water repellant, when molded surface of the molded product of the invention is subjected to a water repelling treatment by fluorine resin, organosilicon compound or the like, it is possible to give water proofing property and, besides the above, various additives such as aromatic agent, antifungal substance, antibacterial agent and coloring agent may be appropriately added thereto if necessary within such an extent that property of the molded product is not deteriorated.

The above surfactant includes surfactant containing anion, cation, nonion and amphoteric ion. However, when it is used, nonionic surfactant is preferred.

Similarly, ethylene oxide, ethylene glycol, propylene oxide, propylene glycol and a polymer containing the same are also useful as additives.

With regard to the above nonionic surfactant, polyoxyethylene alkyl ether, alkylphenol-ethylene oxide adduct such as castor oil-ethylene oxide adduct and nonylphenol or octylphenyl ethylene oxide adduct, higher alcohol phosphate, etc. are listed.

Specific examples of other surfactant are surfactant such as sodium dodecylsulfate and sodium dodecylbenzenesulfonate and silicone.

One of them may be used solely or a mixture comprising two or more thereof may be used. It is also possible to use a commercially available synthetic detergent containing the same.

With regard to the above antifoaming agent, common pH-adjusting agent such as sodium polyphosphate and also the agent used in this field are used.

With regard to the above aggregate, anything may be used so far as it is useful for making the heat generating composition porous and examples thereof are silica-alumina powder, silica-magnesia powder, kaolin, colloidal silica, pumice stone, silica gel, silica powder, mica powder, clay, talc, powder and pellet of synthetic resin and foamed synthetic resin such as foamed polyester and polyurethane.

With regard to the above foaming agent, anything may be used so far as it generates gas and is able to make foams. There are a foaming agent of a decomposition type where it is a single substance which is decomposed by heating to generate gas and a foaming agent of a reaction type where gas is generated by the reaction of two or more substances. Although there is no particular limitation for a foaming agent of a decomposition type, a foaming agent of an inorganic decomposition type is preferably used. Representative examples thereof are sodium bicarbonate, ammonium carbonate, ammonium bicarbonate and ferrous carbonate. It is also able to be used for an object that, during the use as a heat generating body, foams are generated and gas permeability and moisture permeability are enhanced. It is able to be appropriately selected whether heating is conducted for foaming.

With regard to the above moisturizer, anything may be used so far as it is able to moisturize and examples thereof are glycerol and urea.

With regard to the above fibrous material, it is inorganic fibrous material and/or organic fibrous material. Natural fiber such as rock wool, glass fiber, carbon fiber, asbestos fiber, boron fiber, alumina fiber, metal fiber, pulp, paper, nonwoven fabric, woven fabric, cotton and linen; regenerated fiber such as Rayon; semi-synthetic fiber such as acetate; synthetic fiber; and disintegrated product thereof may be used.

With regard to the above binder, examples thereof are sodium silicate, sodium alginate and polyvinylacetate emulsion.

With regard to the water-soluble macromolecular compound, starch, acacia, methyl cellulose (MC), carboxymethyl cellulose (CMC), carboxymethyl cellulose sodium, polyvinyl alcohol, gelatin, hydrolyzed gelatin, polyacrylic acid, polyacrylate, partially neutralized product of polyacrylic acid, polyacrylic acid starch, polyvinylpyrrolidone, hydroxypropyl cellulose, hydroxypropyl methyl cellulose, methylcellulose, carmellose sodium, carboxyvinyl polymer, methoxyethylene maleic acid anhydride copolymer, N-vinylacetamide copolymer, etc. being either solely or in combination of two or more thereof are exemplified.

With regard to the above thickener, those which are commonly used as thickeners such as corn starch and bentonite are listed as examples.

With regard to the above excipient, those which are commonly used as excipients such as casein sodium are listed as examples.

With regard to the above aggregating agent, those which are commonly used as aggregating agents such as corn syrup and mannitol syrup are listed as examples.

With regard to the above soluble pressure-sensitive adhesive material, those which are commonly used as excipients such as polyvinylpyrrolidone are listed as examples.

In the invention, there is no particular limitation for the quality of material and the package constitution of a container bag so far as it is constituted from a substrate material and a covering material, the mixture is able to be retained inside the bag, leakage of the materials during the use of a heat generating body does not take place, sufficient strength having no anxiety of breakage of the bag is available and gas permeability necessary for heat generation is available. Further, there is no particular limitation for size and shape of the heat generating body and that may be flat rectangle, circle, trapezoid, etc. depending upon the place and object for use. Incidentally, besides the substrate material and covering material, the container bag may be also constituted from packing materials including a spreading material.

With regard to the air permeable position of the container bag, any position may be adopted so far as air goes around enough to the heat generating composition. The side facing to a body, the side not facing a body, and the side substantially in parallel with a body are exemplified.

The above packing material is usually constituted from substrate material, covering material, spreading material, etc. There is no particular limitation therefor so far as they are the materials which are used for the heat generating body of this type including a single layer comprising foaming or non-foaming film or sheet and a one where plural layers are layered in the thickness direction.

With regard to the above packing material, it is preferred to have at least one or more property/properties such as non-gas-permeability, gas-permeability, non-shrinking property, shrinking property, non-heat sealing property and heat sealing property.

Further, in the invention, although there is no limitation for sealing of surrounding part and installing the sections in the heat generating composition in a container bag, an example is that a seal layer is formed on at least one of the above substrate material, covering material and spreading material using a pressure-sensitive adhesive and/or heat seal material which is an adhesive of a hot melt type and sealing is carried out at the surrounding of a heat generating composition intervened between at least the substrate material and the covering material by means of thermal fusion (heat seal). Incidentally, when a hot melt type adhesive layer and a hot melt type adhesive layer coexist in the sealed part and sealing is conducted by heat seal, the substantial seal is heat seal whereby this seal is a heat seal.

There is no particular limitation for size and shape of the exothermic bag and that may be in a form of flat rectangle, circle, trapezoid, etc. depending upon the place and the object for use. Besides the above, a purse-shape may be also used.

Although thicknesses of the above substrate material, covering material and spreading material are greatly different depending upon the use, there is no particular limitation therefor.

To be more specific, the above is preferably 5 to 5,000 μm, more preferably 10 to 500 μm and, still more preferably, 20 to 250 μm.

When film thickness of the packing material is less than 5 μm, necessary mechanical strength is not achieved and, further, it may be difficult to make the film thickness uniform whereby that is not preferred.

When film thickness of the packing material is more than 5,000 μm, flexibility lowers, compatibility to the surface of the heat generating body significantly lowers, follow-up property to deformation and movement of the surface of the heat generating body lowers, texture is bad because of roughness and thickness of the whole heat generating body becomes too thick whereby that is not preferred.

It is also possible that unevenness is formed on at least one of substrate material, covering material, spreading material and heat generating composition so as to prevent their movement and deviation. Thus, at the contacting part to at least to the heat generating composition in the substrate material and/or covering material, unevenness is physically formed on the surface thereof when the surface of the substrate material and/or covering material is flat where binding property to the heat generating composition may be enhanced due to such an unevenness to prevent movement and deviation. It goes without saying that the heat generating composition may be uneven.

The packing material of the above container bag may be either in a single-layered structure or a multi-layered structure. Although there is no particular limitation for the structure thereof, examples of the multi-layered structure are that where the substrate material comprises two layers of layer A/layer B or three layers of layer C/layer D/layer E and that where the covering material comprises two layers of layer F/layer G or three layers of layer H/layer I/layer J or layer K/layer L/layer J.

Layer A and layer C are thermoplastic film such as polyethylene; layer B is nonwoven fabric of thermoplastic resin such as nylon, non-water-absorptive paper or water-absorptive paper; layer D is pressure-sensitive adhesive layer, non-water-absorptive paper or water-absorptive paper; layer E is releasing paper or thermoplastic resin film such as polyethylene; layer F, layer H and layer L are porous film or perforated film made of thermoplastic resin such as polyethylene; layer G and layer J are nonwoven fabric of thermoplastic resin of nylon and polyether; layer I is non-water-absorptive paper or water-absorptive paper; and layer K is nonwoven fabric made of thermoplastic resin such as polyethylene.

There is no limitation for the layering method for each layer but each layer may be layered via a gas-permeable pressure-sensitive adhesive layer or a laminating agent layer or may be laminated by means of thermal fusion extrusion, etc.

Further, the heat generating body may be equipped with a skidproof layer or a non-spreading pressure-sensitive adhesive layer at least on one side thereof so as to simplify the installation in use or to prevent movement or deviation of the heat generating body. It is also possible that, when a skidproof layer or a pressure-sensitive adhesive layer is equipped, a releasing paper may be laminated as a protection until it is actually used.

Each of the above substrate material, covering material, spreading material and pressure-sensitive adhesive layer may be any of transparent, non-transparent, colored and non-colored ones. It is also possible that, among the layers constituting each of the materials and layers, a layer which constitutes at least one layer may be in a different color from other layers.

Among the layers constituting the heat generating body, a spreading material is between a substrate material and a covering material and the same material as that for the substrate material and the covering layer may be used.

The heat generating body prepared as such is tightly sealed and stored in a non-gas-permeable bag or the like so as not to contact to oxygen in the air until it is used.

Gas permeability of the container bag is able to be achieved by the use of a gas permeable packing material in one or both side (s) of the bag. There is no particular limitation for the gas permeable packing material constituting the container bag having air permeability. For example, that where at least one of gas permeable film, nonwoven fabric, etc. is layered on paper so as to give gas permeability, that where fine pores are perforated on a non-gas-permeable film such as polyethylene film using a needle so as to give gas permeability, that where fine pores are perforated on a non-gas-permeable packing material in which polyethylene film is laminated on nonwoven fabric so as to give gas permeability, that which is nonwoven fabric or porous film where fiber is layered and thermally compressed and adhered so as to control gas permeability, that where nonwoven fabric is adhered on a porous film, etc. may be used.

When the gas permeable packing material is used on a part of or one or both side (s) of the container bag, gas permeability of the container bag is achieved.

With regard to the gas permeability, there is no particular limitation so far as heat generation is maintained and, in the use as common heat generating body, moisture permeability by Lyssy method is preferably 50 to 10, 000 g/m$^2$.24 hr and, more preferably, 100 to 5,000 g/m$^2$.24 hr.

When the moisture permeability is less than 50 g/m$^2$.24 hr, an exothermic amount becomes small and a sufficient heating effect is not achieved whereby that is not preferred while, when it is more than 10,000 g/m$^2$.24 hr, exothermic temperature becomes high and there is a possibility of causing a problem in view of safety whereby that is not preferred.

In some uses however, there is no limitation for exceeding 10,000 g/m$^2$.24 hr or, in some cases, for using moisture permeability near an open system.

With regard to a non-gas-permeable material constituting the non-gas-permeable part of the above bag, there is no particular limitation so far as it is not gas permeable and examples thereof are film, sheet and coated substance comprising the above hydrophobic polymer or synthetic resin such as polyethylene, polypropylene, nylon, acrylate, polyester, polyvinyl alcohol and polyurethane.

With regard to film having a particularly high non-gas-permeability, that where thin film of metal or metal compound is formed on film of a non-gas-permeable material may be exemplified.

Examples thereof are metal such as silicon, aluminum, titanium, tin, indium, indium-tin, born and zirconium and alloy and mixture containing those metals. Examples of the metal compound are oxide, nitride and acid nitride of those metal and alloy and mixture containing those metals. Semiconductor substances are also included in the metal.

The above metal compound may be anything so far as it is a layer formed from a thin film of metal compound having one layer comprising one member selected from the group consisting of metal oxide, metal nitride and metal acid nitride or a multi-layered structure of two or more layers comprising one or more member(s) selected therefrom.

Examples thereof are a silicon oxide layer, layers of silicon oxide/aluminum oxide, layers of silicon acid nitride/ silicon oxide and layers of silicon nitride/silicon acid nitride/silicon oxide. In that case, $0 < xa \leq 2$, $0 < xb \leq 1.5$, $0 < ya \leq 4/3$ and $0 < yb \leq 1$ when silicon oxide, aluminum oxide, silicon nitride, aluminum nitride, silicon acid nitride and aluminum acid nitride are represented by $SiO_{xa}$, $AlO_{xb}$, $SiN_{ya}$, $AlN_{yb}$, $SiO_{xa}N_{ya}$, $AlO_{xb}N_{yb}$, respectively.

Combination of the type of silicon oxide, aluminum oxide, etc. as such and layer numbers may be free. Between the layers, a component where each of the components including a substrate material is freely mixed or made to react may be present continuously or in an intermittently changing manner.

Another example is that en elongated polyolefin film is layered thereon.

Those are useful as non-gas-permeable film for outer bag which is a non-container bag having air permeability used for storage and transportation of heat generating body and is also able to be used as a substrate material or a covering material.

With regard to the fiber constituting the above woven fabric, it is possible to use natural fiber, regenerated fiber, semi-synthetic fiber, synthetic fiber using natural material such as viscose fiber and a mixture of two or more of them.

With regard to the non-water-absorptive material constituting the non-water-absorptive part of the container bag, there is no particular limitation so far as it is not water-absorptive and examples thereof are film, sheet and painted substance comprising synthetic resin such as polyethylene, polypropylene, nylon, acrylate, polyester, polyvinyl alcohol and polyurethane and the above hydrophobic polymer, etc.

In the invention, it is desirable that the substrate material and the covering material are formed by an extendable film or sheet or, particularly, film or sheet which is able to expand and contract whereby they are more preferably applied to curved area, expanding/contracting area and bending area and, further, more easily follow such shrinking/ elongating area and bending area.

With regard to the above packing material having expanding/contracting property, there is no particular limitation so far as it has an expanding/contracting property. Thus, it is acceptable if it has an expanding/contracting property as a whole and may be a single substance or a compounded substance where expanding/contracting substrate materials or an expanding/contracting substrate material and a non-expanding/contracting substrate material are combined.

Examples thereof are a single substance such as natural rubber, synthetic rubber, elastomer and expanding/ contracting shape memory polymer, a mixed substance or mixed woven product thereof with non-expanding/contracting substrate material, textile, film, spandex yarn, yarn, cord, flat plate, ribbon, slit film, foamed product and nonwoven fabric constituted from their combination and compounded expanding/contracting material by layering of them or of that with non-expanding/contracting one.

Thickness of the above gas-permeable film and non-gas-permeable film is 5 to 500 μm and, preferably, 10 to 350 μm.

With regard to the nonwoven fabric, single nonwoven fabric of single fiber or compounded fiber comprising materials such as rayon, nylon, polyester, acrylate, polypropylene, vinylon, polyethylene, polyurethane, cupra, cotton, cellulose and pulp, mixed woven product of such fibers or layered product of different fiber layers may be used. In terms of a manufacturing method, it is possible to use dry nonwoven fabric, wet nonwoven fabric, spun bond and spun lace. Nonwoven fabric comprising a compounded fiber of a core-sheath structure may be used as well.

Basis weight of nonwoven fabric is preferably 10 to 200 g/m$^2$. When it is less than 10 g/m$^2$, strength cannot

be expected while, even when it is more than 200 g/m$^2$, that is unnecessary in terms of strength.

With regard to the above paper, there is no particular limitation so far as it is non-water-absorptive and examples thereof are non-water-absorptive paper and thick paper. Specific examples are one- or two- or more layered products which are non-water-absorptive selected from thin paper such as blotting paper, tissue and crepe paper; packaging paper such as graft paper; miscellaneous paper such as card paper; corrugated cardboard; inner wick for corrugated cardboard such as pulp wick and special wick; liner for corrugated cardboard such as kraft and jute; thick paper such as coated cardboard; and construction paper such as gypsum board original paper.

If desired, the above paper may be subjected to a waterproof treatment or may be perforated using laser, needle or the like so as to adjust or to give gas permeability.

With regard to the above foamed sheet, an example is a sheet which is formed from at least one member selected from foamed polyurethane, foamed polystyrene, foamed ABS resin, foamed polyvinyl chloride, foamed polyethylene and foamed polypropylene.

With regard to the above hot melt type adhesive, there is no particular limitation so far as it is able to be adhered by heating.

Examples of the hot melt type adhesive as such are adhesive sheet formed by ethylene type hot melt resin such as ethylene-vinyl acetate copolymer resin and ethylene-acrylate copolymer resin (e.g., ethylene-isobutyl acrylate copolymer resin) and hot melt type resin such as polyamide type hot melt resin, polyester type hot melt resin, butyral type hot melt resin, cellulose derivative type hot melt resin, polymethyl methacrylate type hot melt resin, polyvinyl ether type hot melt resin, polyurethane type hot melt resin, polycarbonate type hot melt resin, vinyl acetate and vinyl chloride-vinyl acetate copolymer.

The hot melt type resin may also be compounded with various antioxidants.

With regard to the above hot melt type pressure-sensitive adhesive, anything may be used so far as it is able to be subjected to melt blow, shows viscosity at ambient temperature and is able to be subjected to melting upon heating.

Examples thereof are styrene type elastomer such as SIS, SBS, SEBS and SIPS, acrylate type elastomer having alkyl ester of acrylic acid, methacrylic acid, etc. as a component, olefin type elastomer such as polyethylene, ultra-low-density polyethylene, polypropylene and ethylene-vinyl acetate copolymer and urethane type elastomer. Each of them may be used solely or two or more thereof may be used by blending.

Incidentally, when an olefinic type elastomer is added to a styrene type elastomer, tack or strength is able to be adjusted. In the preparation of a pressure-sensitive adhesive substance, it is possible to compound with an appropriate additive such as tackifier, softener and aging preventer if necessary.

With regard to a pressure-sensitive adhesive for the above pressure-sensitive adhesive layer, there is no particular limitation so far as it has a fixing ability whereby fixing due to pressure-sensitive adhesive force is possible and various forms including acrylate type, urethane type, rubber type, silicon type, polyisoprene type, polyisobutylene type, styrene-isobutyrene-styrene (SIS) type, styrene-isoprene type and polyacrylate type in solvent type, aqueous type, emulsion type, hot melt type, reactive type and pressure-sensitive type thereof may be used.

Examples thereof are vinyl acetate type pressure-sensitive adhesive (vinyl acetate type emulsion and ethylene-vinyl acetate resin type hot melt pressure-sensitive adhesive), polyvinyl alcohol type pressure-sensitive adhesive, polyvinyl acetal type pressure-sensitive adhesive, vinyl chloride type pressure-sensitive adhesive, acrylate type pressure-sensitive adhesive, polyamide type pressure-sensitive adhesive, polyethylene type pressure-sensitive adhesive, cellulose type pressure-sensitive adhesive, chloroprene (neoprene) type pressure-sensitive adhesive, nitrile rubber type pressure-sensitive adhesive, polysulfide type pressure-sensitive adhesive, butyl rubber type pressure-sensitive adhesive, silicone rubber type pressure-sensitive adhesive and styrene type pressure-sensitive adhesive (such as styrene type hot melt pressure-sensitive adhesive).

In case a pressure-sensitive adhesive substance where a substance which radiates far-infrared ray is kneaded with a pressure-sensitive adhesive is prepared, anything may be kneaded with the above pressure-sensitive adhesive without particular limitation for the type of the pressure-sensitive adhesive.

With regard to jell constituting the above jell layer, there is no particular limitation. An example thereof is an aqueous gel layer constituted from aqueous gel.

Examples of the constituting component therefor are polyacrylate such as sodium polyacrylate and partially neutralized product of polyacrylic acid; polyvalent metal such as dry aluminum hydroxide; polyhydric alcohol such as glycerol and propylene glycol; higher hydrocarbon such as light liquid paraffin and polybutene; primary alcohol fatty acid ester such as isopropyl myristate; silicon-containing compound such as silicone oil; fatty acid glyceride such as monoglyceride; oily component such as plant oil such as olive oil; antiseptic such as methyl p-hydroxybenzoate and propyl p-hydroxybenzoate; dissolving agent such as N-methyl-2-pyrrolidone; thickener such as carboxymethyl cellulose; extender such as kaolin; the already-mentioned surfactant such as polyoxyethylene hydrogenated castor oil and sorbitan fatty acid ester; oxycarboxylic acid such as tartaric acid; excipient such as light silicic acid anhydride, the already-mentioned water-absorptive polymer, the already-mentioned water-soluble polymer and kaolin; moisturizer such as D-sorbitol; stabilizer such as sodium edetate, p-hydroxybenzoate and tartaric acid; cross-linking agent such as metal salt of metasilicic acid aluminic acid; and water, etc. In addition to an aqueous gel layer constituted from any combination of the above, a pressure-sensitive adhesive layer where a water-absorptive polymer is further compounded with the above pressure-sensitive adhesive layer or, in other words, a pressure-sensitive adhesive layer used is formed from hot melt type macromolecular substance, alicyclic petroleum resin, softener and water-absorptive polymer absorbs or adsorbs body fluid such as sweat and secretion from the skin and always kept the outer skin surface clean whereby that is preferred from the viewpoint of hygiene.

In the invention, there is no particular limitation for the compounding rate for the composition in a jell layer and a preferred one is 3 to 8% of polyacrylate, 1 to 50% by weight of polyhydric alcohol, 0.03 to 0.15% by weight of polyvalent metal, 1 to 5% by weight of oil component, 3 to 20% by weight of water-soluble polymer, 0.5 to 10% by weight of excipient, 5 to 30% by weight of moisturizer, 0.05 to 8% by weight of stabilizer, 0.03 to 5% by weight of cross-linking agent, 0.5 to 12% by weight of water-absorptive polymer, 5 to 45% by weight of hot melt type macromolecular substance, 5 to 60% by weight of alicyclic petroleum resin, 5 to 60% by weight of softener and 5 to 80% by weight of water.

A particular example is that made from 3 to 8% by weight of polyacrylate, 1 to 15% by weight of polyhydric alcohol, 0.03 to 0.15% by weight of polyvalent metal, 1 to 5% by weight of oil component, 0.5 to% by weight of water-absorptive polymer and 60 to 85% by weight of water; that made from 3 to 20% by weight of water-soluble polymer, 0.5 to 10% by weight of excipient, 5 to 30% by weight of moisturizer, 0.05 to 8% by weight of stabilizer, 0.03 to 5% by weight of cross-linking agent and 30 to 60% by weight of water; and that made from 10 to 35% by weight of hot melt type macromolecular substance, 10 to 55% by weight of alicyclic petroleum resin, 15 to 50% by weight of softener and 1 to 10% by weight of water-absorptive polymer.

If desired, absorption promoter, antiseptic, antioxidant, plasticizer and emulsifier may be added thereto.

An example thereof is an aqueous gel where a mixture comprising 9.2% by weight of partially neutralized product of polyacrylic acid, 5.5% by weight of carmellose sodium, 4.1% by weight of polyvinyl alcohol, 36.6% by weight of glycerol, 36.6 by weight of D-sorbitol liquid, 5.5% by weight of kaolin, 2.2% by weight of tartaric acid, 0.2% by weight of sodium edetate and 0.15% by weight of metal salt of metasilicic acid aluminic acid and pure water are homogeneously mixed. Amount of pure water may be decided upon desire.

There is no particular limitation for thickness of the pressure-sensitive adhesive layer or jell layer and it is preferably 5 to 1,000 μm, more preferably 10 to 500 μm and, still more preferably, 15 to 250 μm. When thickness of the pressure-sensitive adhesive layer is less than 5 μm, there are cases where the desired pressure-sensitive adhesive force is not achieved while, when it is more than 1, 000 μm, the product becomes bulky and not only usability becomes bad but also economy becomes bad whereby that is not preferred.

Although the pressure-sensitive adhesive layer or jell layer is usually formed on the whole surface, it is also possible that polymer having various forms such as net, stripe or dot is intermittently formed so that any of redness, pain, etc. does not happen even when it is used for long time.

At least one member selected from the group consisting of the above substrate material, spreading material, covering material, pressure-sensitive adhesive layer, jell layer and antiskid layer may contain or carry at least one member selected from additional components comprising moisturizer, negative ion-generating substance, bamboo carbon, pyroelectric substance, far-infrared ray-radiating substance, active aromatic compound, inactive aromatic compound, sanitary agent and a mixture thereof.

Particularly when fixation is done to at least one of outside surfaces of the heat generating body such as to a part of the human body, it is advantageous that a side which contacts to the skin either directly or indirectly via clothing or the like is installed with a sanitary agent layer where a sanitary agent is contained in at least one member selected from pressure-sensitive adhesive layer, jell layer and antiskid layer.

With regard to the above sanitary agent, anything may be used so far as it has an effect as a sanitary agent such as drug or pharmaceutical component. Examples thereof are perfume, medicinal plant, herb, Chinese crude drug, intracutaneously absorptive drug, pharmaceutically active substance, aromatizing agent, cosmetic lotion, milky lotion, poultice, antifungal agent, antibacterial agent, bactericide, deodorizer or deodorant and magnetic material.

With regard to the above intracutaneously absorptive drug, there is no particular limitation so far as it is intracutaneously absorptive and examples thereof are skin stimulant, analgesic/anti-inflammatory agent such as salicylic acid and indomethacin, drug acting on central nerve (hypnotic/sedative, antiepileptic agent and neuropsychiatric agent), diuretic, hypotensive drug, coronary vasodilator, antitussive/expectorant, antihistaminic drug, agent for arrhythmia, cardiotonic agent, adrenal cortex hormone agent and local anesthetic. Each of those drugs may be used solely or two more thereof may be used jointly upon necessity.

With regard to the drug, specific examples thereof are blood circulation promoter such as acidic mucopolysaccharide, chamomile, horse chestnut, ginkgo, hamamelis extract, vitamin E, nicotinic acid derivative and alkaloid compound; improving agent for swelling such as horse chestnut, flavone derivative, naphthalenesulfonic acid derivative, anthocyanidin, vitamin P, marigold, choncolitic acid, silanol and *Terminalia, visnaga,* Mayus; agent for making human body slim such as aminophylline, tea extract, caffeine, xanthine derivative, inositol, dextransulfate derivative, horse chestnut, aescin, anthocyanidin, organoiodine compound, hypericum, meadowsweet, field horsetail, rosemary, *Panax ginseng,* common ivy, thiomucase and hyaluronidase; analgesic agent such as indomethacin, dl-camphor, ketoprofen, ginger extract, red pepper extract, piroxicam, felbinac, methyl salicylate and glycol salicylate; and perfume such as lavender, rosemary, citron, juniper, peppermint, eucalyptus, bergamot, yiang, rosewood and orange. One or more thereof may be used.

With regard to the amount of the drug, there is no particular limitation so far as it is within such a range that a pharmaceutical effect can be expected. In view of pharmacological effect, economy, pressure-sensitive adhesive force, etc. however, amount of the intracutaneously absorptive drug to 100 parts by weight of a pressure-sensitive adhesive is preferably 0.01 to 25 part (s) by weight and, more preferably, 0.5 to 15 part(s) by weight.

With regard to antibacterial agent, bactericide or antifungal agent in the invention, there is no particular limitation so far as it expresses aseptic action or bactericidal action or is effective for ringworm such as athlete's feet and specific examples thereof are fatty acid type substance such as phenolic acid derivative, salicylic acid, boric acid, bleaching powder, iodine preparation, heavy metal compound, invert soap, acetic acid and undecylenic acid; salicylic acid type substance; thianthol type substance; tar type substance; mercury type substance such as phenylmercuric acetate; sulfur; antibiotic substance; Polik; Dampa; and Athletan.

With regard to other examples of deodorizer or deodorant in the invention, they may be such things that a smell component is chemically decomposed either by oxidation or reduction and examples thereof are as follows.

Thus, they are those where a decomposing agent such as an element of a platinum group or a compound thereof is contained in a drying agent such as aluminum oxide, silicon oxide, magnesium oxide, titanium oxide, silica gel, zeolite and active carbon or other carrier.

Other examples of the deodorizer or deodorant in the invention are those where aromatic agent is used for setting off the smell.

The heat generating body of the invention is able to give various shapes, thicknesses and temperature zones and, therefore, it is able to be used for various uses such as for indirect *moxa* cautery, inside of shoes such as for feet, joint, making the face beautiful, eye, hot poultice, medical body warmer, neck, waist, mask, glove, hemorrhage, shoulder, cushion, aroma, abdomen, insecticide by evaporation, oxygen absorption and treatment of cancer in addition to common warming of human body.

Examples

Now, the invention will be specifically illustrated by way of the following Examples although the invention shall not be limited thereto.

(Example 1)

Water was added to a mixture of 100 parts by weight of iron powder (DKP manufactured by Dowa Teppun; particle size: not more than 250 µm), 8 parts by weight of active carbon (SA-Super manufactured by Norit; particle size: not more than 300 µm), 4 parts by weight of sodium chloride, 0.15 part by weight of water-absorptive polymer (KI-gel 201K manufactured by Kuraray; particle size: not more than 850 µm), 3.0 parts by weight of wood powder (particle size: not more than 300 µm) and 0.15 part by weight of calcium hydroxide (particle size: not more than 300 µm) followed by subjecting to further mixing to prepare a heat generating composition where water mobility value was 10.

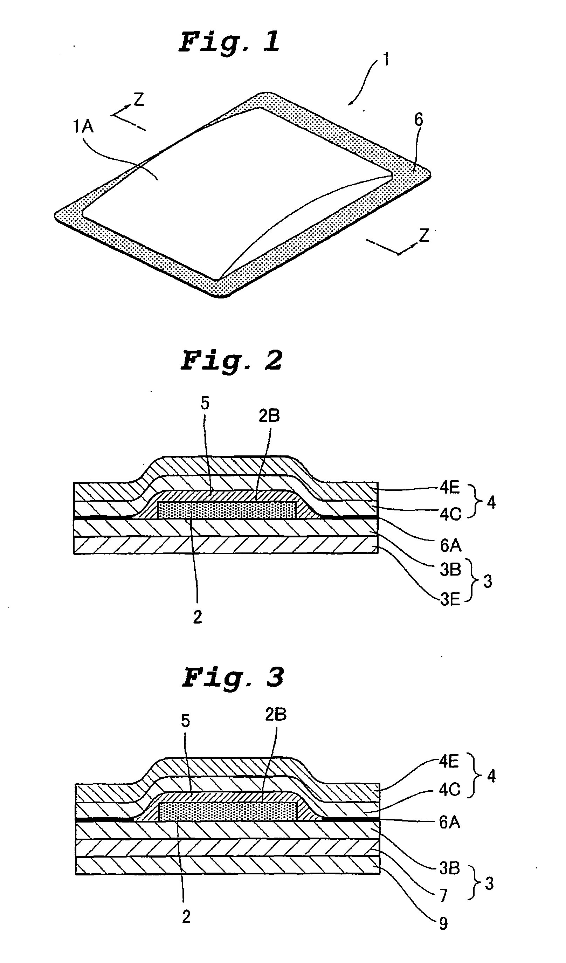

A non-gas-permeable packing material where polyethylene film 3B was laminated on nonwoven fabric 3E was named a substrate material 3 and, on the polyethylene film 3B, the heat generating composition 2 was layered by means of a molding passing through a die using a trimming die having trimming holes in a rectangular shape of 1.7 mm thickness, 115 mm length and 80 mm width to prepare a layered product 2B of a heat generating composition. Further, a hot melt type pressure-sensitive adhesive layer 5 was formed thereon by means of a melt blow method, then a gas-permeable packing material where nonwoven fabric 4E made of nylon and porous polyethylene film 4C were layered in that order was used as a covering material 4 and piled so that surface of the polyethylene film 3B and surface of the porous polyethylene film 4C were faced each other and the surroundings were heat-sealed and cut to prepare a rectangular flat heat generating body 1 (Fig. 1 and Fig. 2) having 135 mm length, 100 mm width and 8 mm seal width. Incidentally, 1A in the drawings shows an exothermic part of the heat generating body 1.

Even when the trimming die was separated from the layered product, shape of the layered product was not deformed, crumbled pieces of the layered product to the surroundings of the layered product were not noted, mixing of the crumbled pieces of the layered product to a sealed part did not take place, sealing was able to be conducted perfectly and poor sealing was not happen as well.

Gas permeability of the covering material 4 in terms of moisture permeability was 400 g/m$^2$.24 hr.

The heat generating body was tightly sealed in a non-gas-permeable outer bag and allowed to stand at room temperature for 24 hours. After 24 hours, the heat generating body was taken out from the outer bag and an exothermic test was conducted whereupon the temperature rose to 36°C within 1 minute and time for maintaining the temperature at not lower than 36°C was as long as 8 hours.

(Example 2)

A non-gas-permeable and non-water-absorptive packing material where polyethylene film was laminated on a non-water-absorptive waterproof paper was used as a substrate material and the heat generating composition prepared in Example 1 was layered on the resulting water-proof paper by a molding by passing through a die using a trimming die whereupon a layered heat generating composition product was prepared. Further, a hot melt type pressure-sensitive adhesive was formed thereon by a melt blow method then, using a gas-permeable packing material where nonwoven fabric made of nylon, kraft paper and porous polyethylene film were layered in that order, layering was conducted in such a manner that the surface of the waterproof paper and the surface of the porous polyethylene film were contacted each other and the surroundings were adhered with compression and cut to prepare a rectangular flat heat generating body having 135 mm length, 100 mm width and 8 mm seal width.
Gas permeability of the covering material in terms of moisture permeability was 400 g/m$^2$.24 hr.
The heat generating body was tightly sealed in a non-gas-permeable outer bag and allowed to stand at room temperature for 24 hours. After 24 hours, the heat generating body was taken out from the outer bag and an exothermic test was conducted whereupon the temperature rose to 36°C within 1 minute and time for maintaining the temperature at not lower than 36°C was as long as 8 hours.

(Comparative Example 1)

A heat generating composition where water mobility value was 30 was prepared according to the same compounding as in Example 1 except water. After that, the same operation as in Example 1 was conducted to prepare a heat generating body having 135 mm length, 100 mm width and 8 mm seal width.
The heat generating body was tightly sealed in a non-gas-permeable outer bag and allowed to stand at room temperature for 24 hours. After 24 hours, the heat generating body was taken out from the outer bag and an exothermic test was conducted but the temperature did not reach 36°C.

(Comparative Example 2)

A mixture was prepared by the same manner as in Example 1 except that 1.5 parts by weight of CMC as a thickener or a binder was added to the compounding of Example 1 except water. To that was added water whereupon a heat generating composition where water mobility value was 10 was prepared. After that, the same operation as in Example 2 was conducted to prepare a heat generating body having 135 mm length, 100 mm width and 8 mm seal width.
The heat generating body was tightly sealed in a non-gas-permeable outer bag and allowed to stand at room temperature for 24 hours. After 24 hours, the heat generating body was taken out from the outer bag and an exothermic test was conducted but the temperature did not reach 36°C.

(Comparative Example 3)

A heat generating composition where water mobility value was 0 or less was prepared in the same compounding as in Example 1 except water. After that, the same operation as in Example 1 was conducted to prepare a heat generating body having 135 mm length, 100 mm width and 8 mm seal width.
When the trimming die was separated from the layered product, loss of shape of the layered product happened and, particularly, surroundings of the layered product lost the shape, crumbled pieces of the layered product came into the area which is to be sealed and poor sealing was resulted at the sealed part.

(Example 3)

As shown in Fig. 3, a substrate material 3 where an acrylate pressure-sensitive adhesive layer 7 was formed on polyethylene film 3B was used instead of the substrate material 3 of Example 1 and the same operation as in Example 1 was conducted to prepare a heat generating body having 135 mm length, 100 mm width and 8 mm seal width. Incidentally, 9 in the drawing shows a releasing film.

(Example 4)

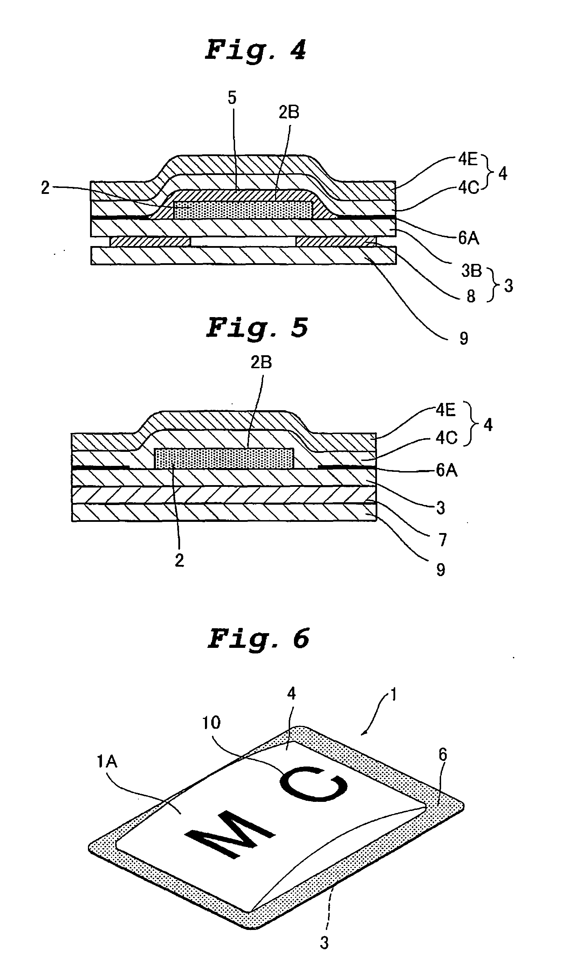

As shown in Fig. 4, a substrate material 3 where an antiskid layer 8 of foamed polyurethane was formed on polyethylene film 3B was used instead of the substrate material 3 of Example 1 and the same operation as in Example 1 was conducted to prepare a heat generating body having 135 mm length, 100 mm width and 8 mm seal width. Incidentally, 9 in the drawing shows a releasing film.

(Example 5)

Water was added to a mixture of 0.3 part by weight of water-absorptive polymer (polyacrylate type; particle size: not more than 64 μm), 8.0 parts by weight of active carbon (particle size: 300 μm), 4.0 parts by weight of sodium chloride, 0.15 part by weight of calcium hydroxide (particle size: not more than 200 μm) and 0.3 part by weight of sodium sulfite (particle size: not more than 200 μm) to 100 parts by weight of iron powder (particle size: not more than 250 μm) followed by stirring and mixing to prepare a heat generating composition where water mobility value was 8.
A nonwoven fabric layer (basis weight: 50 g/m$^2$) comprising hydrophobic and heat-sealing polyester fiber and polyethylene fiber and a high-density polyethylene film where thickness of 40 μm were subjected to a sand lamination using a low-density polyethylene resin where thickness was 40 μm and then a pressure-sensitive adhesive layer 7 equipped with a releasing film 9 was formed on the side of the high-density polyethylene film and the resulting non-gas-permeable layered film was used as a substrate material 3.
A polyester nonwoven fabric 4E where basis weight was 30 g/m$^2$ and a porous polyethylene film 4C where thickness was 40 μm were layered from an exposed surface via a gas-permeable hot melt type pressure-sensitive adhesive layer as shown in the drawing and the product was used as a covering material 4. Moisture permeability of the resulting covering material 4 by a Lyssy method was 400 g/m$^2$.24 hr.
After that, a molded heat generating composition having excessive water was subjected to a molding passing through a die using a trimming die having a thickness of 1.5 mm the same as in Example 1 was layered in a rectangular shape of 1.5 mm thickness on the predetermined position of a polyethylene film on the above substrate material 3, then a covering material 4 was covered thereon so that the porous polyethylene film in the covering material 4 contacted to the layered heat generating composition 2B and, at the surrounding of the layered heat generating composition 2B, a sealed part of the substrate material and the covering material were heat-sealed and cut in a predetermined size to prepare a heat generating body as shown in Fig. 5.
Loss in shape of the layer product did not happen, mixing of crumbled pieces of the layered product at the sealed part 6 was not noted and there was no poor seal.
After that, the product was sealed into an outer bag having gas permeability (not shown).
After 24 hours from sealing into the outer bag, each outer bag was broken and subjected to actual use whereupon exothermic temperature rose to about 36°C within about 1 minute and, after that, heat generation of 36 to 41°C took place for about 7 hours.

(Example 6)

A heat generating body equipped with a pressure-sensitive adhesive layer was manufactured by the same manner as in Example 4 except that the substrate material 3 of Example 4 was changed to a substrate material 3 equipped with a hot melt type styrene type pressure-sensitive adhesive layer as shown in the drawing and that the covering material 4 was changed to a covering material 4 having a design 10 (Fig. 6).
Loss in shape of the layer product did not happen, mixing of crumbled pieces of the layered product at the sealed part 6 was not noted and there was no poor seal.
An exothermic test was also conducted and the same result as in Example 4 was achieved.

(Example 7)

In a nitrogen atmosphere, a mixture of the compounding of Example 1 excluding water was mixed and the air contained therein was substituted with nitrogen. After that, nitrogen-substituted water was added and the mixture was stirred and mixed in a nitrogen atmosphere to prepare a heat generating composition having a water mobility value of 22. Then, nitrogen was blown into the heat generating composition followed by stirring and mixing in a nitrogen atmosphere to prepare a heat generating composition having a water mobility value of 12. By the same manner as in Example 1, a flat rectangular heat generating body of 135 mm length, 100 m width and 8 mm seal width was prepared.
Loss in the shape of the layer product did not happen, mixing of crumbled pieces of the layered product at the sealed part 6 was not noted and there was no poor seal.
Permeability of the covering material 4 in terms of moisture permeability was 400 g/m$^2$.24 hr.

The heat generating body was received tightly sealed in a non-gas-permeable outer bag and allowed to stand at room temperature for 24 hours. The heat generating body was taken out from the outer bag after 24 hours and an exothermic test was conducted whereupon temperature reached 36°C within 1 minute and time for maintaining the heat generation of not lower than 36°C was as long as 8 hours.

(Example 8)

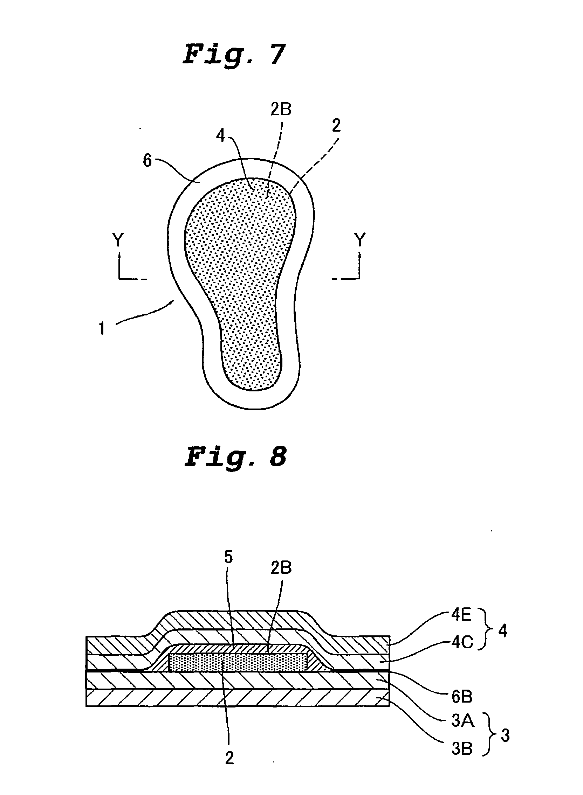

A heat generating body for warming the feet as shown in Fig. 7 in plane figure was prepared. The heat generating body was a heat generating body 1 in a form of a whole foot where a layered product 2B of a heat generating composition 2 was sandwiched between a substrate material 3 and a covering material 4 and, from the surrounding to the outer side of the heat generating composition 2, the substrate material 3 and the covering material 4 were sealed in a width of 10 mm.

Fig. 8 shows its cross-sectional schematic view. With regard to the substrate material 3, a non-water-absorptive waterproof paper 3A of 100 $\mu$m thickness and a polyethylene layer 3B being manufactured by matallocene catalyst and being non-gas-permeable and non-water-permeable is formed in a thickness of 50 $\mu$m. In the substrate material 3, a molding by passing through a die was conducted in such a manner that a layered product 2B of the heat generating composition 2 was directly contacted to the non-water-absorptive waterproof paper 3A.

In the covering material 4, a polypropylene nonwoven fabric 4E having a basis weight of about 80 g/m$^2$ was limited to a porous film 4C having a thickness of 50 $\mu$m and its moisture permeability was 1,000 g/m$^2$.2 hr.

On the substrate material 3, the heat generating composition 2 used in Example 1 was layered at the side of the non-water-absorptive waterproof paper 3A of the substrate material 3 by a molding by passing through a die using a trimming die of 1.7 mm thickness and then a hot melt type SIS type pressure-sensitive adhesive layer 5 was formed thereon by a melt blow method. After that, the side of the porous film 4C of the covering material 4 was attached to the pressure-sensitive adhesive layer 5 and passed through a press roll to make the layered product 2B of the heat generating composition 2 about 1.5 mm and the surrounding of the layered product 2B of the heat generating composition 2 was sealed by compression by means of a pressure adhering seal bar of 120°C to prepare a heat generating body for warming the feet in a whole foot type.

Even when the trimming die was separated from the layer product, loss in the shape of the layer product did not happen, crumbled pieces of the layered product to the surrounding of the layered product was not resulted, mixing of crumbled pieces of the layered product at the sealed part 6 was not noted and there was no poor seal provided that seal was conducted completely.

The above heat generating body for foot warming was sealed into a non-gas-permeable bag, allowed to stand for 24 hours, taken out therefrom by breaking the non-gas-permeable bag and used by placing in leather shoes whereupon an excellent effect of maintaining the heat was achieved for not shorter than 6 hours.

(Example 9)

A heat generating body was prepared using a heat generating composition of Example 1, a gas-permeable covering material 4 having 600 g/m$^2$.day of moisture permeability which was formed by laminating a porous film on a polyamide type nonwoven fabric of basis weight of 40 g/m$^2$ via a gas-permeable pressure-sensitive adhesive layer in a nonwoven fabric form of basis weight of 7 g/m$^2$ and a substrate material having a pressure-sensitive adhesive layer where an SIS type hot melt type SIS type pressure-sensitive adhesive layer having a thickness of 30 $\mu$m formed on a separator was adhered on one side of polyethylene film together with the separator.

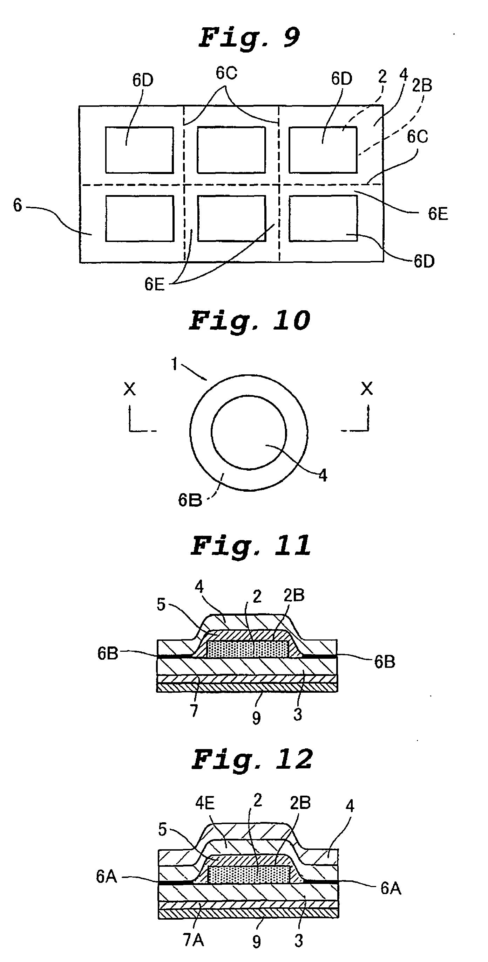

Firstly, a predetermined amount of heat generating composition 2 was layered on polyethylene film of the substrate material by means of a molding by passing through a die using a trimming die having six-section regions having three longitudinal lines and two transverse lines where each one section region 6D was 1.7 mm thickness, 30 mm length and 25 mm width, the layered product 2B was covered so as to contact to the side of porous film of the covering material 4, surrounding of each layer product 2B was heat-sealed 6A and the sectioning part 6E was subjected to a perforating process (resulting in a broken line by which cutting by hand is possible) to prepare a heat generating body of 130 mm length and 80 mm width where each seal width was 10 mm (Fig. 9).

Loss in the shape of the layer product did not happen, mixing of crumbled pieces of the layered product at the sealed part 6 was not noted and there was no poor seal.

The heat generating body was sealed in a non-gas-permeable, stored, allowed to stand for 24 hours, taken out therefrom by breaking the non-gas-permeable bag and used whereupon an excellent heating effect was achieved for not shorter than 6 hours. Its close contact to human body was good and, during the use, there was no detachment due to warp of the packing material.

(Example 10)

Fig. 10 and Fig. 11 each is an exothermic body 1 equipped with an SIS type pressure-sensitive adhesive layer 7 (thickness: 35 microns) where seal width is 10 mm in which a layered product 2B of heat generating composition of exothermic part 2 has 35 mm diameter and thickness 2 mm. In this example, a heat generating composition 2 was layered on a substrate material 3 by a molding by passing through a die to form a layered product 2B of a heat generating composition of 3 mm thickness. After that, a hot melt type SIS type gas-permeable pressure-sensitive adhesive layer 5 was formed on the layered product 2B of heat generating composition and the substrate material 2 and further covered by a gas-permeable covering material 4 and compressed to make the layered product 2B of the heat generating composition 2 mm and a sealed part was sealed by compression 6B and cut in a circular shape to prepare a circular heat generating body comprising the constitution of releasing film 9/pressure-sensitive adhesive layer 7/substrate material 3/layered product 2B of heat generating composition/gas-permeable pressure-sensitive adhesive layer 5/covering material 4. This was sealed into a non-container bag having air permeability which did not permeate the air. After 24 hours, the circular heat generating body in a form of the non-container bag having air permeability was taken out and subjected to an exothermic test whereupon heat of 43°C was generated during 1 hour.

Fig. 12 shows an example where, in the heat generating body of Fig. 11, nonwoven fabric made of polyethylene 4E which is a kind of a spreading material was formed between the gas-permeable pressure-sensitive adhesive layer and the covering material so that heat sealing 6A was able to be done by the polyethylene nonwoven fabric 4 and, further, a pressure-sensitive adhesive layer was changed to a jell layer 7A.

(Example 11)

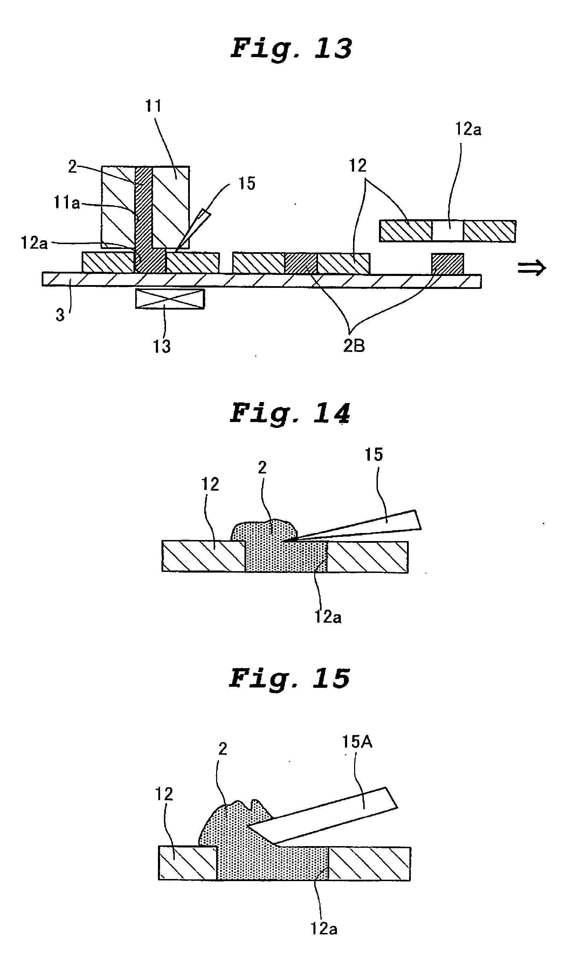

Fig. 13 and Fig. 14 each shows an example of a molding passing through a die using a scraper 15.

Thus, a substrate material in a form of roll film having 130 mm width is horizontally sent, in a thickness of 1 mm, at a predetermined speed between a dice 11 and a magnet 13 located upper and lower surface, respectively together with a die 12 for molding where a desired shape is punched at the center of the die. The heat generating composition 2 of the invention is sent from the upper surface of the die 12 to a die hole 12a via a hole 11a of the dice 11. The heat generating composition 2 is cut in the same surface as that of the die 12 by a scraper 15 which is placed at the front in the moving direction and, at the same time, received in a die hole 12a whereupon a shape in 1.5 mm thickness is formed on the substrate material 3. After that, the die 12 is removed to give a molded product layered on the substrate material 3.

After that, although not shown in the drawing, a viscous polymer of a styrene-isoprene-styrene block copolymer (SIS) type is formed in a form of net by a melt blow method on the surface of the molded product, a covering material is covered thereon and the surrounding of the molded product region is sealed by heat sealing and cut in a predetermined shape whereupon a heat generating body having a desired shape is prepared. Further, the cut heat generating body of the invention is sent to a packing step and sealed in an air-tight outer bag. The same molding is also possible even when the above scraper 15 is changed to a pushing scraper 15A. Fig. 14 shows a scraper 15 while Fig. 15 shows a pushing scraper 15A. Incidentally, so far as a function of a pushing scraper is maintained, it is possible to conduct any deformation such as that the front end of the pushing scraper is trimmed to make it round.

(Example 12)

The heat generating composition of Example 1 was used and, using a non-gas-permeable packing material where polyethylene was laminated on nonwoven fabric, the heat generating composition was layered on the polyethylene by a molding by passing through a die using a trimming die having a rectangular trimming hole of 1.7 mm thickness, 115 mm length and 80 mm width whereupon a layered product of the heat generating composition was prepared. After that, a non-gas-permeable packing material in which nonwoven fabric made of nylon and porous film were layered in that order was used as a covering material and piled so that polyethylene side and porous side were contacted each other and the surrounding was heat-sealed and cut to prepare a rectangular flat heat generating body of 135 mm length, 100 mm width and 8 mm seal width.

Even when the trimming die was separated from the layer product, loss in the shape of the layer product did not happen, crumbled pieces of the layered product to the surrounding of the layered product was not resulted, mixing of crumbled pieces of the layered product at the sealed part 6 was not noted and there was no poor seal provided that seal was conducted completely.

Gas-permeability of the covering material in terms of moisture permeability was 400 g/m$^2$.24 hr.

The heat generating body was received in a non-gas-permeable outer bag by tightly sealing and allowed to stand at room temperature for 24 hours. After 24 hours, it was taken out from the outer bag and an exothermic test was conducted whereupon temperature reached not lower than 36°C within 1 minute and time for maintaining the heat generation was as long as 8 hours.

Industrial Applicability

1) In a heat generating composition of the invention, an exothermic substance, a reaction promoter, water and a carbon component are essential components, water mobility value thereof is 20 or less, maximum particle size of water-insoluble solid components excluding the reaction promoter and water is 1 mm or less and 80% or more thereof has a particles size of 300 μm or less whereby its molding property, shape-holding property and exothermic characteristic are excellent and, accordingly, it is possible to provide a heat generating body having an excellent exothermic characteristic and having various shapes from thin to thick types and from rectangle to even that having curves such as circle.

2) In a product where a molded product of a heat generating composition of the invention is appropriately compressed by pressure, loss of the shape hardly happen and perforated film is able to be used even when perforated film having difficult pressure adjustment is used instead of porous film as a material for gas-permeable part and even when inner pressure of a container bag becomes identical with outer pressure and, accordingly, range for selection of gas-permeable material becomes wide, reduction in cost is possible and a substance to be heated is able to be homogeneously heated at appropriate temperature for long time.

3) Since it is a heat generating composition having excessive water, fluidity, molding property and shape-holding property are significantly high as compared with the conventional powdery heat generating composition and, for example, it is able to be uniformly layered on a substrate material which is sent at high speed at a predetermined range continuously and correctly by means of molding by passing through a die, printing, etc. and is further able to manufacture various shapes such as rectangle and circle from ultra-thin type to thick type.

4) Since it is a heat generating composition having excessive water, sprinkling of powder of heat generating composition to the surrounding does not happen unlike the conventional case and it is possible to conduct a factory control which completely fulfills the GMP standards in the manufacture of medical instruments and pharmaceuticals in future.

As mentioned above, it is now possible to prepare a heat generating composition and a heat generating body having excellent characteristics which have not been available in the conventional heat generating composition and heat generating body.

## Claims

1. In a heat generating composition which generates heat by contacting with air, a heat generating composition which is **characterized in that** an exothermic substance, a reaction promoter, water and a carbon component are essential components, water mobility value thereof is 20 or less, maximum particle size of water-insoluble solid components excluding the reaction promoter and water is 1 mm or less where 80% or more thereof has a particle size of 300 μm or less, water in the heat generating composition does not function as a barrier layer and exothermic reaction takes place when contacted to the air.

2. The heat generating composition according to claim 1, wherein particle size of all of the above water-insoluble solid components is 300 μm or less.

3. The heat generating composition according to claim 1, wherein the heat generating composition uses a heat generating composition having a water mobility value of 7 or more as a material and water content is adjusted by a non-oxidative gas.

4. The heat generating composition according to claim 1, wherein the heat generating composition contains at least one member selected from additional components consisting of water-retaining agent, water-absorptive polymer, hydrogen formation inhibitor, pH adjusting agent, surfactant, antifoaming agent, hydrophobic polymer compound, pyroelectric substance, far-infrared ray-radiating substance, negative ion-generating agent, antioxidant, aggregate, heat generating aid, oxidation catalyst, organosilicon compound, fibrous material, sanitary agent, fertilizer component, active aromatic compound, inactive aromatic compound, moisturizer and a mixture thereof.

5. A heat generating body which is **characterized in that** the heat generating composition mentioned in claim 1 is sealed in a container bag where at least a part thereof has air permeability.

6. The heat generating body according to claim 5, wherein the heat generating composition is layered and received in a container bag, the layered heat generating composition forms two or more plural sectional exothermic parts being separately located and an aggregated exothermic part is formed from aggregation of the sectional exothermic parts.

7. The heat generating body according to claim 6, wherein the container bag comprises a substrate material and a covering material, at least one of the substrate material and the covering material has gas permeability and each of the sectional exothermic parts is sectioned by a sectional part by means of a heat seal of the substrate material and the covering material.

8. The heat generating body according to claim 5, wherein the container bag comprises a substrate material and a covering material, at least one of the substrate material and the covering material has gas permeability, the heat generating composition is placed on the substrate material, a pressure-sensitive adhesive layer is further located at least on the heat generating composition and a spreading material is furthermore located thereupon.

9. The heat generating body according to claim 8, wherein at least the heat generating composition is subjected to a compressing treatment in the substrate material, covering material, gas permeable pressure-sensitive adhesive layer, spreading material and heat generating composition.

10. The heat generating body according to claim 7, wherein a perforation is formed on the sectional part.

11. The heat generating body according to claim 5, wherein any one or more of letters, designs, symbols, numerals, patterns, photographs and pictures are formed on at least a part thereof including a releasing paper.

12. The heat generating body according to claim 5, wherein at least a part thereof including a releasing paper is colored.

13. The heat generating body according to claim 7, wherein a pressure-sensitive adhesive layer or a jell layer is layered on at least a part of exposed surfaces of at least one of the substrate material and the covering material.

14. The heat generating body according to claim 13, wherein the pressure-sensitive adhesive layer or the jell layer contains and carries at least one member selected from additional components consisting of moisturizer, negative ion-generating substance, bamboo carbon, pyroelectric substance, far-infrared ray-radiating substance, active aromatic compound, inactive aromatic compound, sanitary agent and a mixture thereof.

15. The heat generating body according to claim 5, wherein the heat generating body is intervened between two non-gas-permeable films or sheets, the two films or sheets are punched into a size which is not smaller than the heat generating body together with or after the intervening and the two films or sheets are adhered by fusion at the surrounding which is more than the size of the heat generating body together with or after the punching.

## *Fig. 1*

## *Fig. 2*

## *Fig. 3*

## Fig. 4

## Fig. 5

## Fig. 6

**Fig. 7**

**Fig. 8**

...

## Fig. 9

## Fig. 10

## Fig. 11

## Fig. 12

## Fig. 13

## Fig. 14

## Fig. 15

EP 1 577 363 A1

## Fig. 16

## Fig. 17

## Fig. 18

29

# Fig. 19

# Fig. 20

## INTERNATIONAL SEARCH REPORT

| International application No. |
| --- |
| PCT/JP03/16338 |

**A. CLASSIFICATION OF SUBJECT MATTER**
Int.Cl$^7$ C09K5/16, A61F7/08

According to International Patent Classification (IPC) or to both national classification and IPC

**B. FIELDS SEARCHED**

Minimum documentation searched (classification system followed by classification symbols)
Int.Cl$^7$ C09K5/16, A61F7/08

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2002-155273 A (Kaoru USUI), | 1-5,13-15 |
| Y | 28 May, 2002 (28.05.02), | 6-10 |
| | Claims 1 to 27; Par. Nos. [0019], [0067], [0075], [0179]; examples 4 to 5, 7 | |
| | (Family: none) | |
| X | JP 2002-60741 A (Showa Denko Kabushiki Kaisha), | 1-5,11-14 |
| Y | 26 February, 2002 (26.02.02), | 6-10 |
| | Claims; Par. Nos. [0041], [0057]; examples 1 to 3 | |
| | (Family: none) | |
| Y | JP 2001-71402 A (Kabushiki Kaisha Hakugen), | 6-10 |
| | 21 March, 2001 (21.03.01), | |
| | Claims; Figs. 1, 3 | |
| | (Family: none) | |

☐ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 26 January, 2004 (26.01.04) | 10 February, 2004 (10.02.04) |

| Name and mailing address of the ISA/ | Authorized officer |
| --- | --- |
| Japanese Patent Office | |
| Facsimile No. | Telephone No. |

Form PCT/ISA/210 (second sheet) (July 1998)